# NEW EUROPEAN PATENT SPECIFICATION

(11) **EP 0 639 229 B2**
(45) Date of publication and mention of the opposition decision: **25.11.2009**
(45) Mention of the grant of the patent: 10.03.1999
(21) Application number: 93909875.2
(22) Date of filing: 30.04.1993
(51) Int. Cl.: C12Q 1/68, C12R 1/32

(54) **RAPID DETECTION OF ANTIBIOTIC RESISTANCE IN MYCOBACTERIUM TUBERCULOSIS**
SCHNELLNACHWEIS DER ANTIBIOTIKARESISTENZ IN MYCOBACTERIUM TUBERCULOSIS
DETECTION RAPIDE DE LA RESISTANCE AUX ANTIBIOTIQUES DE MYCOBACTERIUM TUBERCULOSIS

(30) Priority: 30.04.1992 US 875940; 14.08.1992 US 929206; 17.09.1992 FR 9211098; 16.04.1993 FR 9304545
(43) Date of publication of application: 22.02.1995
(73) Proprietor: INSTITUT PASTEUR, 75724 Paris Cedex 15 (FR); ASSISTANCE PUBLIQUE, HOPITAUX DE PARIS, 75004 Paris (FR); UNIVERSITE PIERRE ET MARIE CURIE, 75252 Paris Cédex 05 (FR)
(72) Inventor: HEYM, Beate, F-75010 Paris (FR); COLE, Stewart, F-92140 Clamart (FR); YOUNG, Douglas, Middlesex HA4 6ED (GB); ZHANG, Ying, London W12 0UE (GB); HONORE, Nadine, F-92700 Colombes (FR); TELENTI, Amalio, CH-3115 Gerzensee (CH); BODMER, Thomas, CH-3423 Ersigen (CH)
(74) Representative: Almond-Martin, Carol
(86) International application number: PCT/EP1993/001063
(87) International publication number: WO 1993/022454

(56) References cited:
- EP-A1- 0 223 156
- WO-A1-91/06674
- JOURNAL OF MOLECULAR BIOLOGY vol. 202, no. 1, August 1988, LONDON pages 45 - 58 JIN ET AL. 'Mapping and sequencing of mutations in the Escherichia coli rpoB gene that lead to rifampicin resistance'
- THE JOURNAL OF GENERAL MICROBIOLOGY vol. 60, no. 1, January 1970, LONDON pages 125 - 132 SRIPRAKASH ET AL. 'Isoniazid-resistant mutants of Mycobycterium tuberculosis H37RV: ...' cited in the application
- NATURE vol. 358, no. 6387, 13 August 1992, LONDON pages 591 - 593 ZHANG ET AL. 'The catalase-peroxidase gene and isoniazid resistance of Mycobacterium tuberculosis' cited in the application
- THE LANCET vol. 341, no. 8846, 13 March 1993, LONDON pages 647 - 650 TELENTI ET AL. 'Detection of rifampicin-resistance mutations in mycobacterium tuberculosis'
- RESEARCH IN MICROBIOLOGY vol. 143, no. 7, September 1992, AMSTERDAM pages 721 - 730 HEYM ET AL. 'Isolation and characterization of isoniazid-resistant mutants of Mycobacterium smegmatis and M. aurum' cited in the application
- The Biology of Mycobacteria, p.354-437, Author Frank G Winder, Ed. C Ratledge & J Stanford, Academic Press London 1982
- ANTIMICROBIAL AGENTS & CHEMOTHERAPY vol. 35, no. 6, 1991,, pages 1035 - 1039
- FEMS MICROBIOLOGY LETTERS vol. 79, 1991,, pages 247 - 250
- MOLECULAR MICROBIOLOGY vol. 9, no. 6, 1993,, pages 1239 - 1246
- Molecular Biology of the Mycobacteria, p.121-138, Author: C.Martin et al., Ed.:J Mc Fadden, Surrey Univerisity Press, 1990
- NATURE vol. 345, 1990,, pages 739 - 743
- ANTIMICROBIAL AGENTS & CHEMOTHERAPY vol. 35, 1991,, pages 1937 - 1939
- FRANK WINDER: 'Mode of Action of the Antimycobacterial Agents and Associated Aspects of the Molecular Biology of the Mycobacteria' THE BIOLOGY OF MYCOBACTERIA vol. 1, 1982, LONDON, pages 354 - 437
- GAYATHRI DEVI ET AL.: 'The purification and properties of peroxidase in M. tuberculosis H37Rv and its possible role in the mechanism of action of isonicotinic acid hydrazide' BIOCHEM. J. vol. 149, 1975, pages 187 - 197

## Description

This invention relates to the rapid detection of strains of Mycobacterium tuberculosis that are resistant to antibiotics, particularly isoniazid, rifampicin and streptomycin. More particularly, this invention relates to a method of detecting antibiotic resistance in Mycobacterium tuberculosis, e.g. either as a result of mutations in the relevant genes or by nucleic acid hybridization. This invention also relates to a nucleic acid probe and a kit for carrying out the nucleic acid hybridization. The invention further relates to the chromosomal location of the katG gene and its nucleotide sequence.

### BACKGROUND OF THE INVENTION

Despite more than a century of research since the discovery of Mycobacterium tuberculosis, the aetiological agent of tuberculosis, by Robert Koch, this disease remains one of the major causes of human morbidity and mortality. There are an estimated 3 million deaths annually attributable to tuberculosis (Snider, 1989), and although the majority of these are in developing countries, the disease is assuming renewed importance in the West due to the increasing number of homeless people and the impact of the AIDS epidemic (Chaisson et al., 1987; Snider and Roper, 1992).

Isonicotinic acid hydrazide or isoniazid (INH) has been used in the treatment of tuberculosis for the last forty years due to its exquisite potency against the members of the "tuberculosis" groups - Mycobacterium tuberculosis, M. bovis and M. africanum (Middlebrook, 1952; Youatt, 1969). Neither the precise target of the drug, nor its mode of action, are known, and INH treatment results in the perturbation of several metabolic pathways. There is substantial evidence indicating that INH may act as an antimetabolite of NAD and pyridoxal phosphate (Bekierkunst and Bricker, 1967; Sriprakash and Ramakrishnan, 1970; Winder and Collins, 1968, 1969, 1970), and other data indicating that the drug blocks the synthesis of the mycolic acids, which are responsible for the acid-fast character of mycobacterial cell walls (Winder and Collins 1970; Quemard et al., 1991). Shortly after its introduction, INH-resistant isolates of Mycobacterium tuberculosis emerged and, on characterization, were often found to have lost catalase-peroxidase activity and to show reduced virulence in guinea pigs (Middlebrook et al., 1954; Kubica et al., 1968; Sriprakash and Ramakrishnan, 1970).

Very recently, INH-resistance has acquired new significance owing to a tuberculosis epidemic in the USA due to multidrug resistant (MDR) variants of M. tuberculosis (CDC, 1990; 1991a, b) and the demonstration that such strains were responsible for extensive nosocomial infections of HIV-infected individuals and health care workers (Snider and Roper, 1992). In view of the gravity of this problem, there exists a need in the art to determine the relationship between INH-resistance and catalase-peroxidase production.

More particularly, there is a need in the art to understand the molecular mechanisms involved in drug sensitivity. In addition, there is a need in the art to develop a simple test permitting the rapid identification of INH-resistant strains. Further, there is a need in the art for reagents to carry out such a test.

Gayathri Devi et al (Biochem J. 1975) describes the purification of a new protein, Y-enzyme, in M. tuberculosis H37Rv, having catalase and peroxidase activities, and discloses that loss of INH uptake, loss of catalase, peroxidase, Y-enzyme activities and INH resistance are linked. No process for detection of such a resistance is disclosed in this document.

Rifampicin too is a major antibiotic used for the treatment of infections by mycobacterium, particularly Mycobacterium tuberculosis and Mycobacterium leprae. Because some mycobacteria grow slowly, possible rapid and efficient tests for the testing of resistance to rifampicin or analogues thereof must be made available. Likewise the invention aims at a rapid detection of strains of Mycobacterium tuberculosis which are resistant to streptomycin. Because of the development of resistance to streptomycin, the latter antibiotic has been used together with other antibiobics, e.g. isoniazid. Thus adequat treatment of tuberculosis should be preceded by rapid and efficient detection of resistances to the three majeur antibiotics, isoniazid, rifampicin and streptomycin.

The disclosure by Winder et al (in "The biology of Mycobacteria, 1982, Eds Ratledge & Stanford, Academic Press, London, vol 1, Chapter 8) relates to the mode of action of rifampicin and streptomycin in mycobacteria, however discloses neither the mechanism of action of these antibiotics, nor a process for detection of resistance to them.
Document Zhang et al (Nature, 1992) discloses the use of mycobacterial genetics to study the basis of INH resistance, but not of rifampicin and streptomycin.

### SUMMARY OF THE INVENTION

Accordingly, this invention aids in fulfilling these needs in the art by providing a process for detecting in vitro the presence of cells of a Mycobacterium tuberculosis resistant to isoniazid and other drugs, such as rifampicin or analogues thereof, and streptomycin.

By analogues of rifampicin, a particularly meant derivatives of 3-formyl-rifamycin, particularly as a result of substitution the rein for the substituant present either in the naphtofuranonyl group or of the site chain at position 7 of the naphtofuranonyl group, or by the introduction or removal of a double band in the lateral chain.

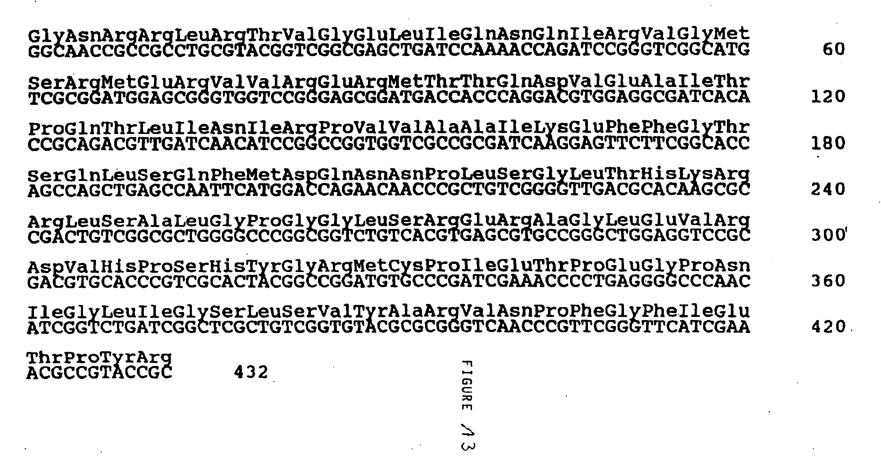

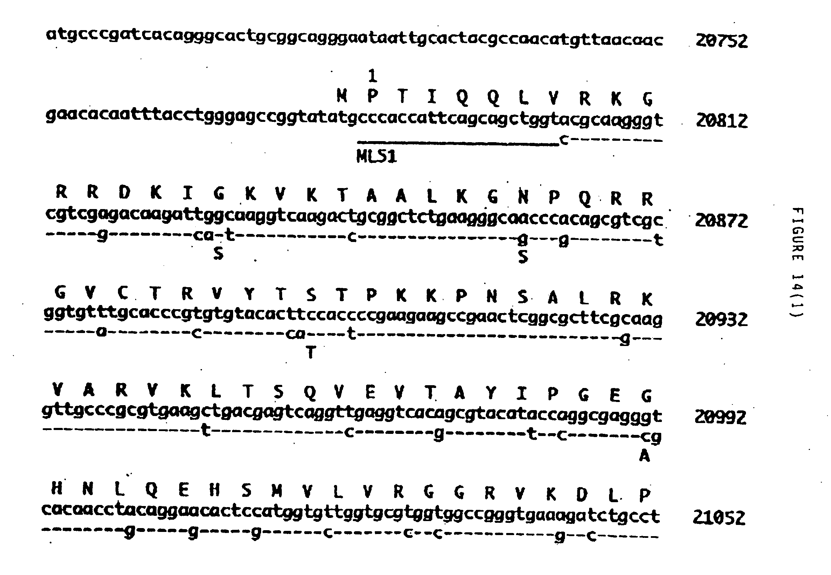

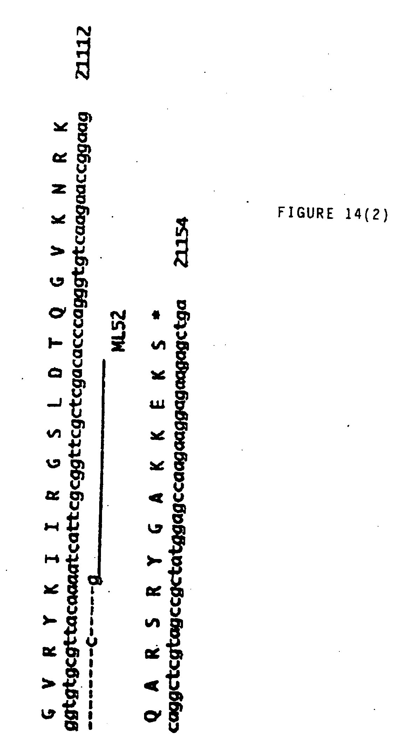

The invention relates to a process for the detection of resistance to an antibiotic in a mycobacterium which comprises detecting a mutation in a gene selected from the group comprising the rpoB gene or fragment thereof which gene or fragment thereof is capable of hybridising with the sequence shown in Figure 13, and the rpsL gene or fragment thereof, which gene or fragment thereof is capable of hybridising with the Mycobacterium tuberculosis sequence shown in Figure 14.

In accordance with the invention, a process for detecting in vitro the presence of nucleic acids of a Mycobacterium tuberculosis resistant to isoniazid, wherein the process comprises the steps of:
- contacting said nucleic acids previously made accessible to a probe if required under conditions permitting hybridization;
- detecting any probe that had hybridized to said nucleic acids;
wherein said probe comprises a nucleic acid sequence, which is 2.5 kb EcoRV-KpnI fragment of plasmid pYZ56 and wherein said fragment contains a BamHI cleavage site, wherein isoniazid-resistant Mycobacterium tuberculosis do not contain DNA which hybridises with this fragment.

For instance, this process comprises the steps of :
(A) depositing and fixing nucleic acids of the cells on a solid support, so as to make the nucleic acids accessible to a probe;
(B) contacting the fixed nucleic acids from step (A) with a probe under conditions permitting hybridization;
(C) washing the filter resulting from step (B), so as to eliminate any non-hybridized probe; and then
(D) detecting any hybridized probe on the washed filter resulting from step (C).
The probe comprises a nucleic acid sequence which is present in a 2.5 kb EcoRV-KpnI fragment of plasmid pYZ56, wherein said fragment contains a BamHI cleavage site. This fragment has been found to be associated with intracellular DNA of isoniazid-sensitive Mycobacterium tuberculosis and is capable of distinguishing such antibiotic sensitive microorganisms from isoniazid-resistant Mycobacterium tuberculosis, which do not contain DNA that hybridizes with this fragment under the conditions described hereinafter.

This invention further provides nucleotide sequences, such as RNA and DNA, of isoniazid-resistant Mycobacterium tuberculosis encoding the region of the katG gene of Mycobacterium tuberculosis that imparts isoniazid sensitivity absent from isoniazid-resistant cells.

This invention also provides a probe consisting of a label, such as a radionuclide, bonded to a nucleotide sequence of the invention.

In addition, this invention provides a hybrid duplex molecule consisting essentially of a probe of the invention hydrogen bonded to a nucleotide sequence of complementary base sequence, such as DNA or RNA.

Also, this invention provides a process for selecting a nucleotide sequence coding for a catalase-peroxidase gene of Mycobacterium tuberculosis, or for a portion of such a nucleotide sequence, from a group of nucleotide sequences, which comprises the step of determining which of the nucleotide sequences hybridizes to a probe of the invention. The nucleotide sequence can be a DNA sequence or an RNA sequence. The process can include the step of detecting a label on the nucleotide sequence.

The invention also discloses compounds obtained as products of the action of the enzyme catalase, or a similar enzyme on isoniazid. The katG gene or a derivative of this gene which retains a similar activity can be used as a source of catalase protein. The new compounds are selected by reactivity on INH-resistant-mycobacterial strains by the antibiogram method such as described in H. David et al.'s "Methodes de laboratoire pour Mycobacteriologie clinique" edited by Pasteur Institute, ISBN N° 0995-2454.

### BRIEF DESCRIPTION OF THE DRAWINGS

This invention will be described in greater detail by reference to the drawings in which:

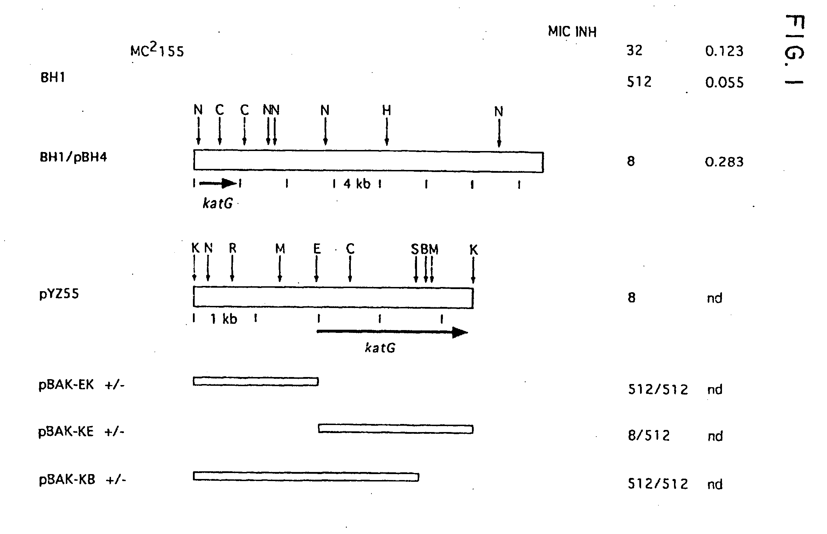

Fig 1. shows the INH-resistant M. smegmatis strain, BH1 (Gayathri et al., 1975) (a derivative of strain MC²-155) was transformed with a pool of M. tuberculosis-H37Rv shuttle cosmids (kindly provided by Dr. W.R. Jacobs, New York) and individual clones were scored for INH-susceptibility. Cosmid pBH4 consistently conferred drug susceptibility and the transformant overproduced catalase (assayed as in Heym, 1992). The restriction map of the DNA insert from pBH4 is shown along with that of the insert from pYZ55 - a plasmid containing katG of M. tuberculosis H37Rv, isolated on the basis of hybridization with an oligonucleotide probe (5'-TTCATCCGCATGGCCTGGCACGGCGCGGGCACCTACCGC-3') designed to match the amino acid sequence from a conserved region of E. coli hydroperoxidase I (HPI). Restriction sites for the following enzymes are indicated : B, BamHl; C, Clal; E, EcoRV; H, HindIII, K, Kpnl; M, Smal; N, Notl; R, EcoRl; S, Sacl. Transformation of BH1 with a mycobacterial shuttle plasmid, pBAK14, Zhang et al., 1991, containing the 4.5 kb insert from pYZ55 similarly conferred INH-susceptibility. MIC's are also shown for BH1 transformed with subfragments derived from pYZ55 and inserted into pBAK14 in one (+) or other (-) orientation. The katG gene and the ability to confer INH-susceptibility both mapped to a 2.9 kb EcoRV-Kpnl fragment (pBAK-KE+).

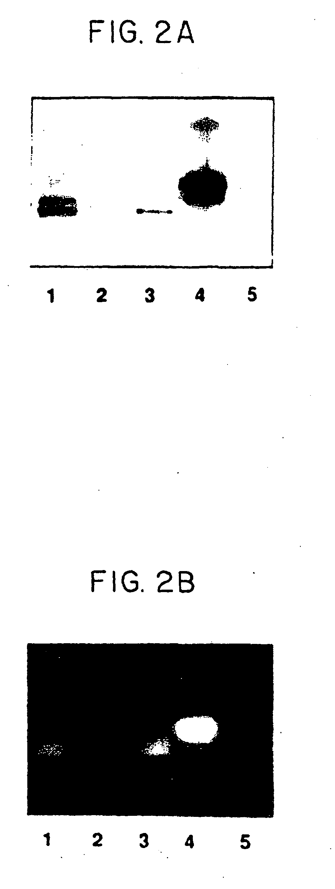

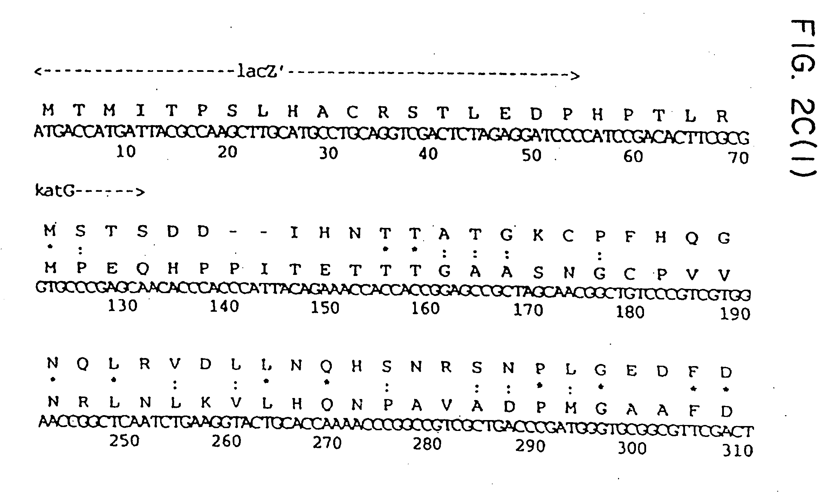

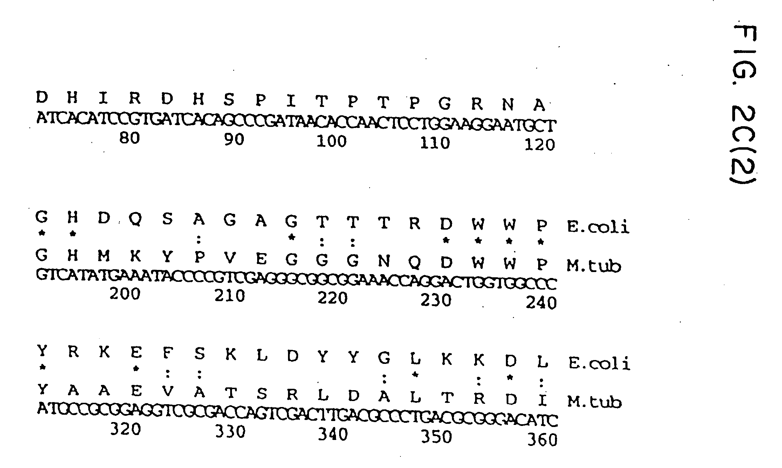

Fig. 2 shows extracts from M. tuberculosis H37Rv and from E. coli strains transformed with a variety of plasmid constructs that were prepared for activity gel analysis as described previously (Zhang et al., 1991). Non-denaturing gels containing 8% polyacrylamide were stained for catalase (panel A) and peroxidase (panel B) activities as described by Wayne and Diaz (Wayne et al., 1986). Lane 1, M. tuberculosis H37Rv; 2, E. coli UM2 (katE, katG; 3, E. coli UM2/pYZ55; 4, E. coli UM2/pYZ56 (the 2.9 kb EcoRV-Kpnl_fragment in pUC19, corresponding to pBAK-KE+ in Fig. 1); 5, E. coli UM2/pYZ57 (pYZ55 with a BamHl-Kpnl deletion, corresponding to pBAK-KB+ in Fig. 1). M. tuberculosis catalase and peroxidase activities migrated as two bands under these conditions (lane 1); the same pattern was seen for the recombinant enzyme expressed by pYZ55 (lane 3). pYZ56 (lane 4) expresses a protein of increased molecular weight due to a fusion between katG and lacZ' from the vector as shown in panel C. Panel C also shows partial sequence alignment with E. coli HPI.

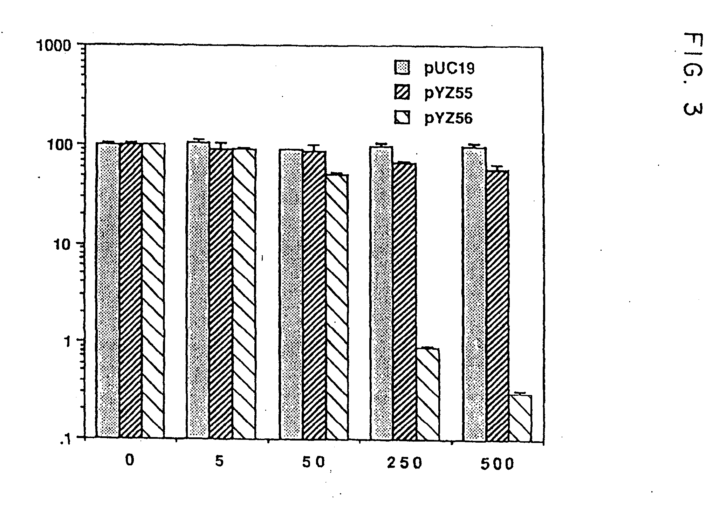

Fig. 3 shows an E. coli strain with mutations in both katG and katE (UM2 Mulvey et al., 1988) that was transformed with pUC19 vector alone, pYZ55 expressing M. tuberculosis katG and pYZ56 with high level expression of M. tuberculosis katG. Overnight cultures in Luria-Bertani broth supplemented with appropriate antibiotics were plated out in the presence of varying concentrations of INH and colony forming units were assessed. Results of a representative experiment are shown with error bars indicating the standard deviation observed in triplicate samples. Overexpression of M. tuberculosis katG similarly conferred susceptibility to high concentrations of INH in E. coli UM255 (katG. katE, Mulvey et al., 1988), but had no effect on catalase-positive strains such as E. coli TG1. In some experiments, high concentrations of INH had detectable inhibitory effect on growth of UM2 and UM255, alone, but in all experiments inhibition of pYZ56-transformants was at least 10-100 fold greater than that observed in the corresponding vector controls.

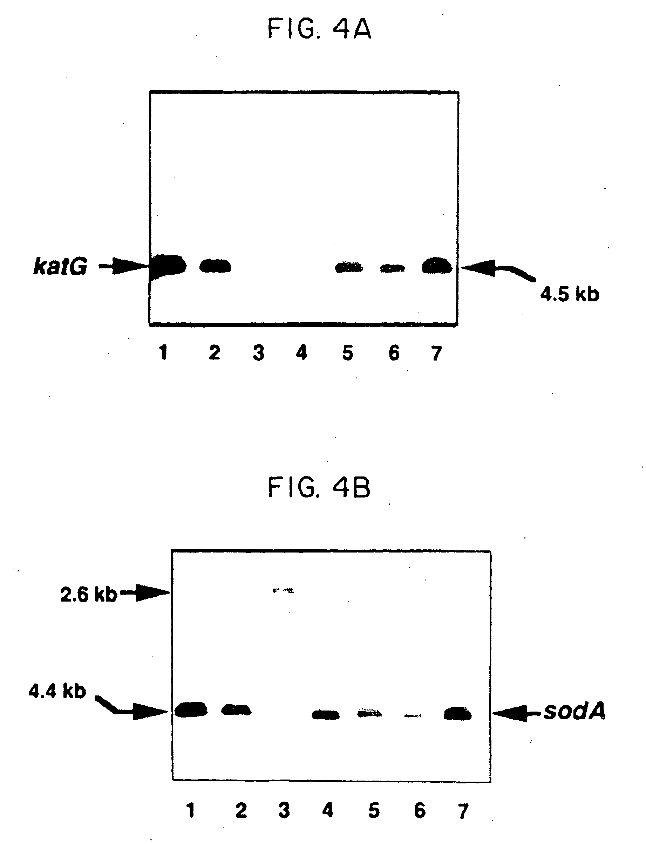

Fig. 4 shows Southern blots prepared using genomic DNA from different M. tuberculosis strains, digested with Kpnl, that were probed with (A) katG (the 4.5 kb Kpnl fragment), and (B) the SOD gene (1.1 kb EcoRl-Kpnl fragment, Zhang et al., 1991). Labelling of probes and processing of blots was performed as described previously (Eiglmeier et al., 1991; Maniatis et al., 1989). Lane 1, H37Rv; 2, strain 12 - MIC 1.6 µg/ml INH; 3, B1453 - MIC > 50 µg/ml INH (Jackett et al., 1978); 4, strain 24 - MIC > 50 µg/ml INH; 5, 79112 - INH-sensitive (Mitchison et al., 1963); 6, 12646 - INH-sensitive (Mitchison et al., 1963); 7, 79665 - INH-sensitive (Mitchinson et al., 1963). INH susceptibilities were confirmed by inoculation of Lowenstein-Jensen slopes containing differing concentrations of INH.

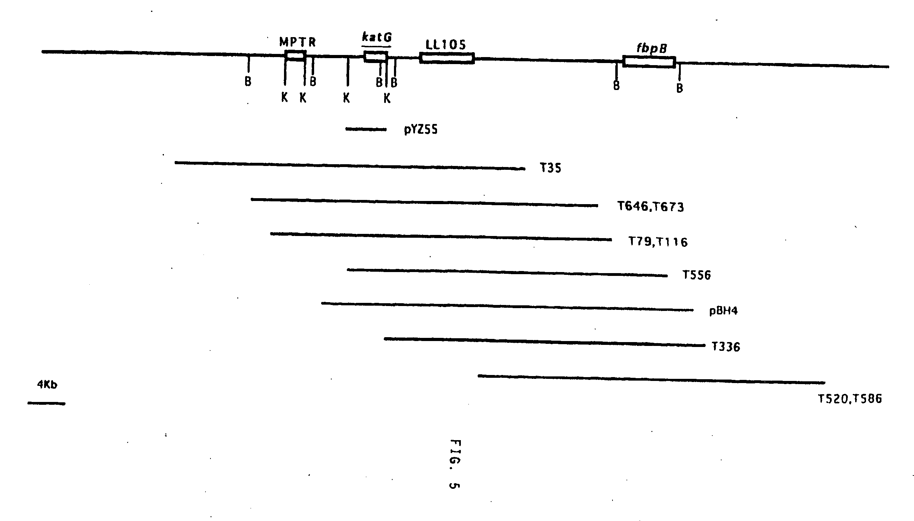

Fig. 5. Organization of the katG locus. The upper bar corresponds to a stretch of the M. tuberculosis chromosome spanning the katG region and the positions of individual cosmids used to construct the map are shown below together with the original shuttle cosmid pBH4 and pYZ55. The locations of some key restriction sites (B, BamHI; K, Kpnl) are shown together with the approximate location of the known genetic markers: fbpB encoding the alpha or 85-B antigen (Matsuo et al., 1988); katG, catalase-peroxidase; LL105, an anonymous λgt11 clone kindly supplied by Å Andersen; MPTR, major polymorphic tandem repeat (Hermans et al., 1992).

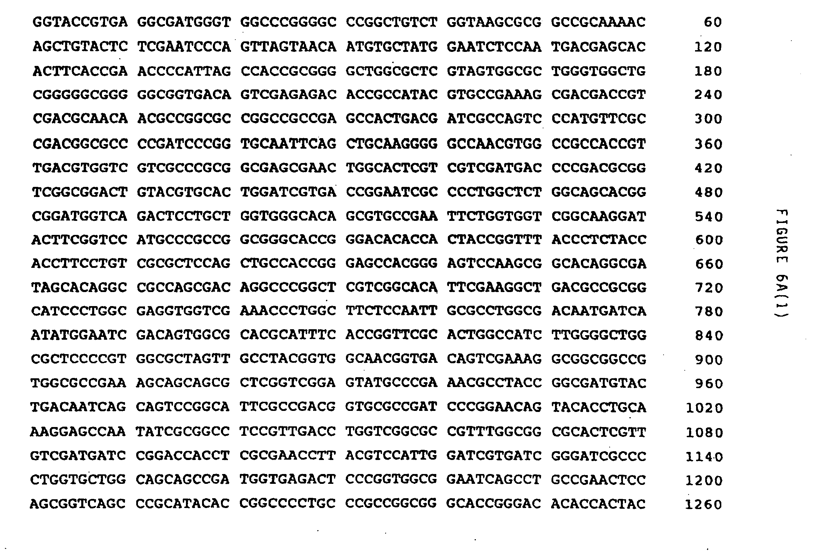

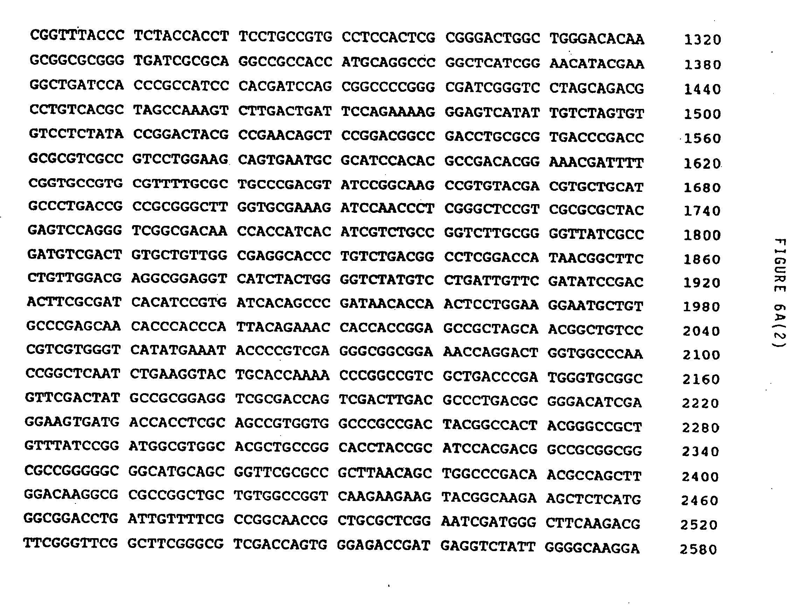

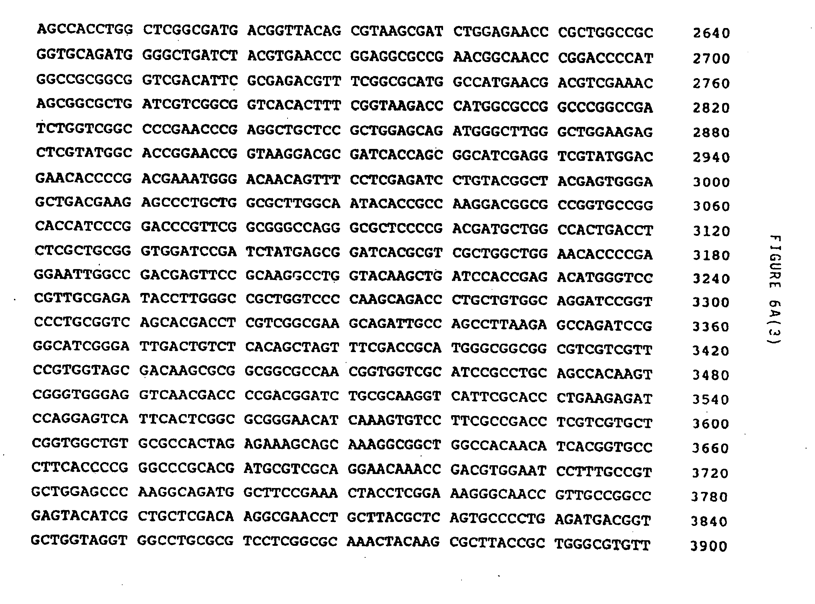

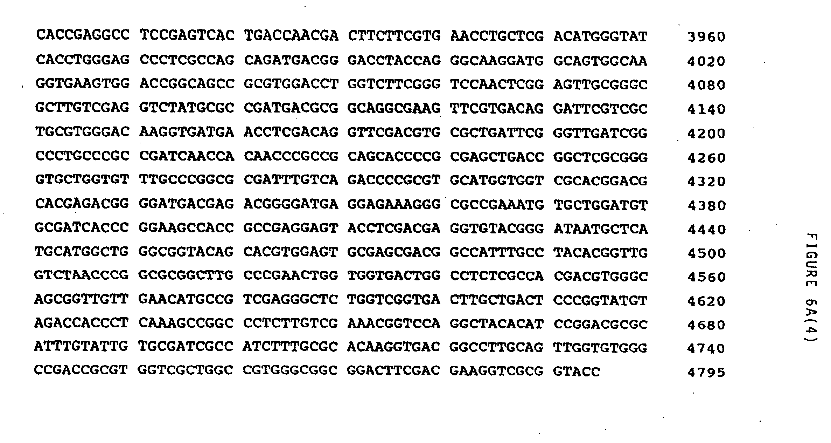

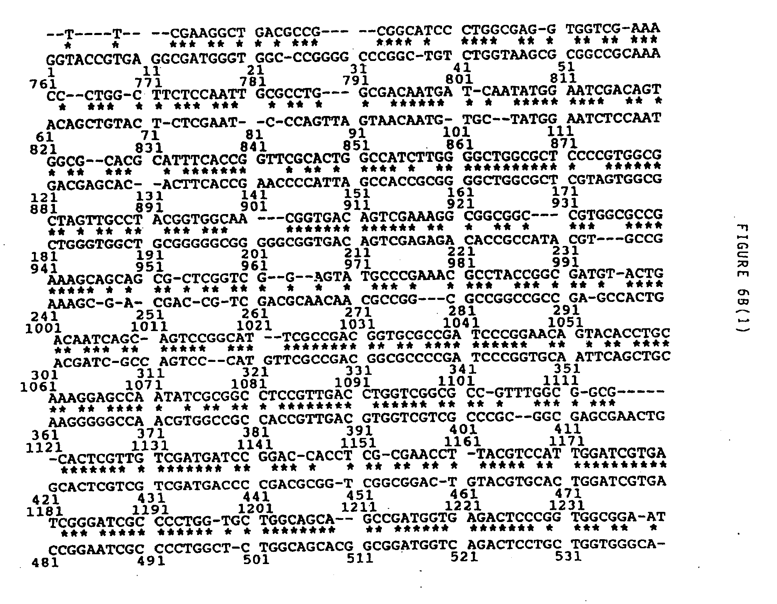

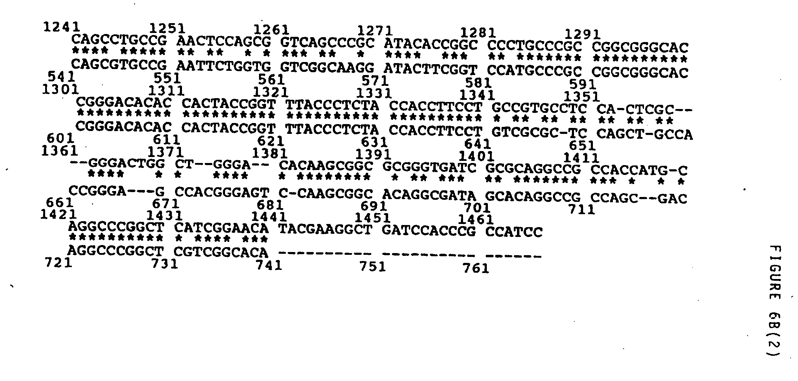

Fig. 6. A. Nucleotide sequence of the KpnI fragment bearing katG. This sequence has been deposited in the EMBL data-library under accession number X68081. The deduced protein sequence is shown in the one letter code. B. Alignment of the two copies of the 700 bp direct repeat with identities shown as * and - denoting pads introduced to optimize the alignment. Numbering refers to the positions in Fig. 2A.

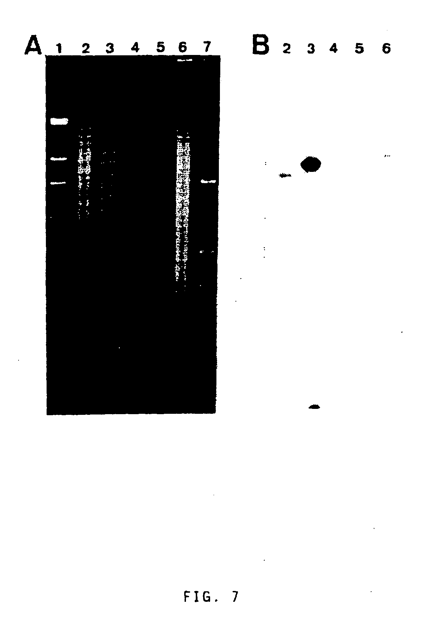

Fig. 7. Distribution of katG in mycobacteria. A. Samples of different bacterial DNAs (1.5 µg) were digested with RsrII, separated by agarose gel electrophoresis and stained with ethidium bromide; lanes 1 and 7, size markers; M. leprae; lane 3, M. tuberculosis H37Rv; lane 4, M. aordonae; lane 5, M. szulgai; lane 6, M. avium. B. Hybridization of the gel in A, after Southern blotting, with a katG specific probe.

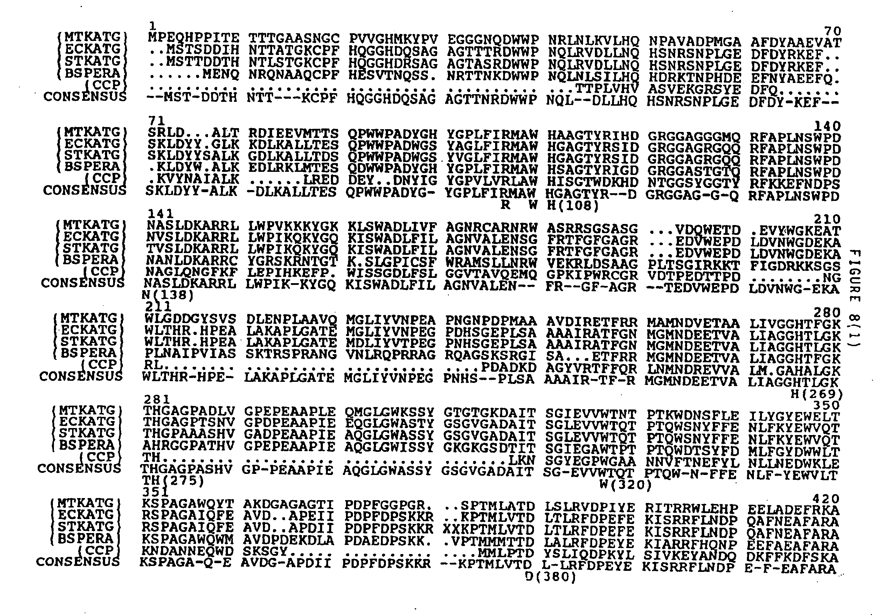

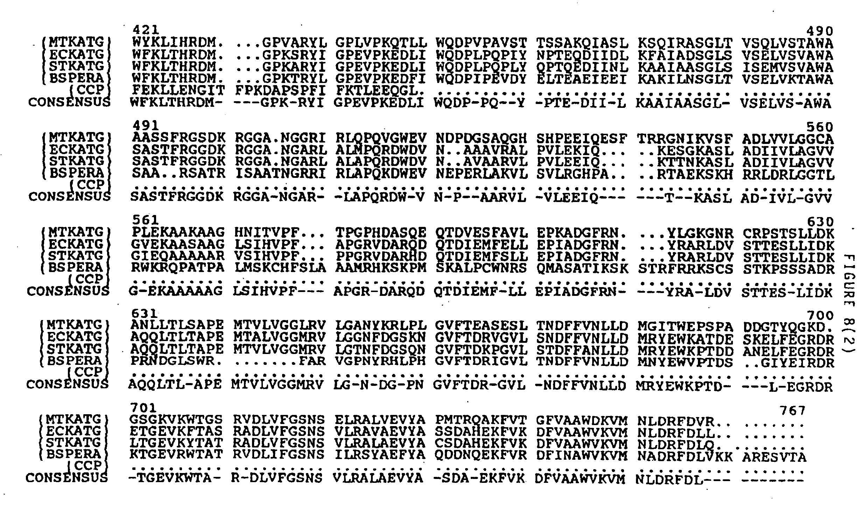

Fig. 8. Primary structure alignment of catalase-peroxidases. The sequences are from M. tuberculosis H37RV, mtkatg; E.coli, eckatg (Triggs-Raine et al., 1988); S. typhimurium, stkatg; B. stearothermophilus, bspera (Loprasert et al., 1988) and yeast cytochrome c peroxidase (ccp; Finzel et al., 1984). The alignment was generated using PILEUP and PRETTY (Devereux et al., 1984) and . denote gaps introduced to maximize the homology. Key residues from the active site and the peroxidase motifs (Welinder, 1991), discussed in the text, are indicated below the consensus.

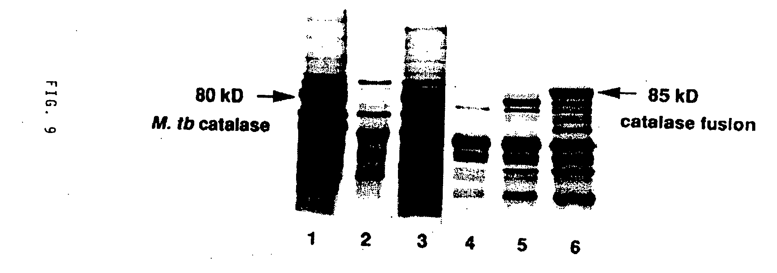

Fig. 9. Western blot analysis of M. tuberculosis KatG produced in different bacteria. Proteins were separated by SDS-polyacrylamide gel electrophoresis then subjected to immunoblotting, and detection with antiserum raised against BCG, as described in Zhang et al., 1991. Lane 1, soluble extract of M. tuberculosis H37Rv; lane 2, M. smeamatis MC²155 harboring the vector pBAK14; lane 3, MC²155 harboring pBAK-KK (katG+); lane 4, E. coli UM2 (katE, katG), lane 5, UM2 harboring pYZ55 (katG⁺); lane 6, UM2 harboring pYZ56 (lacZ'::katG).

### DETAILED DESCRIPTION OF PREFERRED EMBODIMENT

The recent emergence of large numbers of strains of M. tuberculosis showing multidrug resistance in the United States is a most alarming development given the extreme contagiousness of this organism. This danger has been strikingly illustrated by several small tuberculosis epidemics in which a single patient infected with MDR M. tuberculosis has infected both HIV-positive individuals, prison guards and healthy nursing staff (CDC 1990, 1991; Daley et al., 1992; Snider and Roper, 1992). Given the gravity of the current worldwide HIV epidemic, it is conceivable that if AIDS patients in the West, like those in Africa, were to be infected with MDR M. tuberculosis strains (rather than members of the M. avium/M. intracellulare complex) widespread dissemination of the disease would result.

Isoniazid (INH) is a bactericidal drug which is particularly potent against the tuberculosis group of mycobacteria - Mycobacterium tuberculosis, M. bovis, and M. africanum - and, in consequence, it has been particularly effective in the treatment of tuberculosis. Standard anti-tuberculosis regimens generally include INH and rifampicin, often in combination with the weaker drugs, pyrazinamide, ethambutol or streptomycin. Besides its use in therapy INH is also given to close contacts of patients as a prophylactic measure.

INH-resistant mutants of M. tuberculosis, the agent of the human disease, show two levels of resistance: low (1 to 10 µg/ml) and high (10 to 100 µg/ml). INH-resistance is often associated with loss of catalase activity and virulence. Recently, cwing to the AIDS epidemic, increased homelessness and declining social conditions, tuberculosis has reemerged as a major public health problem in developed countries, particularly the USA. An alarming feature of the disease today is the emergence of multiple drug-resistant organisms and rapid nosocomial transmission to health care workers and HIV-infected patients. This has prompted CDC to propose new recommendations for the treatment of multiple resistant strains (at least INH and rifampicin) and the prevention of transmission. To obtain fresh insight into the problem of INH-resistance and to develop a rapid diagnostic test the following study was performed.

Clearly, it is essential to understand the mechanisms of resistance to INH and rifampicin, the main anti-tuberculosis agents, as this will allow novel chemotherapeutic strategies to be developed and facilitate the design of new compounds active against MDR strains.

This invention demonstrates that it is the catalase-peroxidase enzyme, HPI, which is the INH target, and it is suggested that this enzyme alone mediates toxicity. Compelling evidence of this conclusion was obtained by expression of the M. tuberculosis katG gene in a catalase-negative mutant of E. coli as this resulted in this bacterium becoming sensitive to INH. Moreover, the isolation of the M. tuberculosis INH-sensitivity gene, katG, is important as it will facilitate the rapid detection of INH-resistant strains by means of hybridization and PCR-based approaches. The high frequency of katG deletions in clinical strains, as shown here, should simplify this procedure.

### Identification of an M. tuberculosis gene involved in INH-sensitivity

A heterologous approach was employed to isolate M. tuberculosis gene(s) involved in INH-sensitivity. BH1 is a spontaneous mutant of the easily transformable M. smegmatis strain MC²155 (Snapper et al., 1990), that is resistant to 512 µg/ml of the INH and lacks catalase-peroxidase activity (Heym et al., 1992). As there is a strict correlation between INH-sensitivity and these enzyme activities, transformation of BH1 with a plasmid carrying the appropriate gene from M. tuberculosis should lead to their restoration and concomitant INH-sensitivity.

Consequently, DNA was prepared from a pool of M. tuberculosis shuttle cosmids in Escherichia coli and introduced into BH1 by electro-transformation. Over 1000 kanamycin-resistant transformants were then scored for INH-sensitivity, and four clones that failed to grow on medium containing 32 g/ml of INH, the MIC from wild type strain MC²155, were obtained.

After re-transformation of BH1, only one of these, pBH4, consistently conferred the INH-sensitive phenotype. Restriction digests with BamHI, KpnI, NotI, ClaI and HindIII showed the M. tuberculosis chromosomal DNA carried by pBH4 to be about 30 kb in size. A map produced with the last three enzymes is presented in Fig. 1.

When pBH4 was used as a hybridization probe to detect homologous clones in the library, a further eight shuttle cosmids were isolated. On transformation into BH1, five of these (T35, T646, T673, T79, T556) restored INH-sensitivity, and showed similar restriction profiles to pBH4. In particular, a KpnI fragment of 4.5 kb was present in all cases.

Attempts to subclone individual BamHI fragments did not give rise to transformants capable of complementing the lesion in BH1 suggesting that a BamHI site might be located in the gene of interest. In contrast, pBH5, a derivative of pBH4, was constructed by deletion of EcoRI fragments and this showed that a 7 kb segment was not required for restoration of INH-sensitivity.

Transformants harboring shuttle cosmids that complemented the INH-resistant mutation of BH1 were examined carefully and the MICs for several antibiotics were established. In all cases, the MIC for INH had been reduced from 512 to 8 µg/ml, a value lower than that of the sensitive strain MC²155 (32 µg/ml). This hypersensitive phenotype suggested that the recombinant clones might be overproducing an enzyme capable of enhancing INH-toxicity. Enzymological studies showed that these transformants all produced about 2-fold more peroxidase and catalase than the wild type strain MC²155, which is INH-sensitive.

In addition to INH, many MDR-strains of M. tuberculosis are no longer sensitive to rifampicin, streptomycin, ethambutol and pyrazinamide. To examine the possibility that there might be a relationship between resistance to INH and these compounds, the MICs of several drugs for various M. smegmatis strains and their pBH4 transformants were determined, but no differences were found.

### Cloning the M. tuberculosis catalase gene

A 45-mer oligonucleotide probe was designed based on the primary sequences of highly conserved regions in the catalase-peroxidase enzymes, HPI, of E. coli (Triggs-Raine et al., 1989), and Bacillus stearothermophilus (Loprasert et al., 1988). When genomic blots of M. tuberculosis DNA were probed with this oligonucleotide, specific bands were detected in most cases. As KpnI generated a unique fragment of 4.5 kb that hybridized strongly, this enzyme was used to produce a size selected library in pUC19.

Upon screening with the oligonucleotide probe, an appropriate clone, pYZ55, was obtained. A restriction map of the insert DNA is presented in Fig. 1 where it can be seen that this corresponds exactly to part of pBH4. Independent confirmation was also obtained by cross-hybridization.

By means of various subcloning experiments the smallest fragment expressing M. tuberculosis catalase-peroxidase activity in E. coli was found to be a 2.5 kb EcoRV-KpnI fragment which, as expected, contained a cleavage site for BamHI. Partial DNA sequence analysis showed that the katG gene carried by pYZ56 encodes a catalase-peroxidase enzyme that is highly homologous to the HPI enzymes of E. coli and B. stearothermophilus:
M.tuberculosis APLNSWPDNASLDKARRLLWPSKKKYGKKLSWADLIV
E.coli *********V***********I*Q***Q*I*****FI
B.stearothermophilus **********N******C*GR**RNT*T*-*LGPICS
(Fig. 2; Triggs-Raine et al., 1988); (Loprasert et al., 1988). Identical residues are indicated by *. HPI activity was detected in both E. coli and M. smegmatis by staining (see below).

### Catalase-peroxidase involvement in INH-sensitivity

Having cloned the M. tuberculosis katG gene, it was of immediate interest to investigate the genetic basis of the association between catalase-negativity and isoniazid resistance. A series of constructs was established in the shuttle vector pBAK14 and used to transform the INH-resistant M. smegmatis mutant BH1. Only those plasmids carrying a complete katG gene produced HPI and restored INH-sensitivity. The smallest of these, pBAK14, carried a 2.5 kb EcoRV-KpnI fragment thus demonstrating that the 2 kb region upstream of katG was not involved, and that catalase-peroxidase activity alone was sufficient to render mycobacteria susceptible to INH.

Cell-free extracts were separated by non-denaturating polyacrylamide gel electrophoresis and stained for peroxidase and catalase activity. Under these conditions, the M. tuberculosis enzyme gave two bands of peroxidase activity (lane 1) which comigrated with catalase activity (Heym et al., 1992).

When introduced into E. coli, the katG gene directed the synthesis of the same proteins, whereas pYZ56 produced proteins slightly larger in size. This is due to the construction of an in-frame lacZ::katG gene fusion. Activity stains were also performed with cell extracts of M. smegmatis. The presence of the katG gene from the M. tuberculosis in BH1 led to the production of catalase-peroxidase enzyme, which displayed the same electrophoretic mobility as the enzyme made in M. tuberculosis, or in E.coli, and the native HPI of M. smegmatis.

### Basis of INH-resistance in M. tuberculosis

It has been known for many years that a subset of INH-resistant strains, particularly those resistant to the highest drug concentrations, are of lower virulence in the guinea pig and devoid of catalase activity. Genomic DNA was prepared from several clinical isolates of M. tuberculosis and analyzed by Southern blotting using the 4.5 kb KpnI fragment as a probe. In two highly resistant strains, B1453 and 24, the catalase gene has been deleted from the chromosome whereas in others (Fig. 3), such as strain 12, showing low level resistance it is still present but not expressed. Additional studies showed that the region immediately prior to katG was highly prone to rearrangements.

### M. tuberculosis HPI renders E. coli sensitive to INH

To determine whether the HPI enzyme of M. tuberculosis could confer INH sensitivity on E. coli, a series of catalase mutants was transformed with pYZ56 and the MICs determined. Wild type strains were not susceptible to INH, but mutants lacking both endogenous catalase activities, but harboring pYZ56, showed growth inhibition when high levels of INH (500 µg/ml) were present, whereas untransformed strains were insensitive.

For purposes of this invention, a plasmid containing the restriction endonuclease map shown in Fig. 1 was deposited in strain no. 463 with the National Collection of Cultures of Microorganisms (C.N.C.M.) of the Institut Pasteur, in Paris, France on May 18, 1992, under culture collection accession No. I-1209. This plasmid contains the nucleic acid sequence of the invention, namely, the 4.5 kb KpnI-KpnI fragment of plasmid pYZ56 having the BamHI cleavage site in the fragment.

In general, the invention features a method of detecting the presence of isoniazid-resistant Mycobacterium tuberculosis in a sample including providing at least one DNA or RNA probe capable of selectively hybridizing to isoniazid-sensitive Mycobacterium tuberculosis DNA to form detectable complexes said probe comprising a nucleic acid sequence, which is 2,5kb EcoRV-KpnI fragment of plasmid pY256, and wherein said fragment contains a BamHI cleavage site. Detection is carried out with a sample under conditions which allow the probe to hybridize to isoniazid-sensitive Mycobacterium tuberculosis DNA present in the sample to form hybrid complexes and detecting the hybrid complexes as an indication of the presence of isoniazid-sensitive Mycobacterium tuberculosis in the sample. (The term "selectively hybridizing", as used herein, refers to a DNA or RNA probe which hybridizes only to isoniazid-sensitive Mycobacterium tuberculosis and not to isoniazid-insensitive Mycobacterium tuberculosis.) The sample can be comprised of the Mycobacterium tuberculosis cells or a portion of the cells or cell contents enriched in Mycobacterium tuberculosis nucleic acids, especially DNA. Hybridization can be carried out using conventional hybridization reagents. The particular hybridization conditions have not been found to be critical to the invention.

A preferred probe according to this aspect of the invention comprises a nucleic acid sequence which encodes a polypeptide of the formula Ala Pro Leu Asn Ser Trp Pro Asp Asn Ala Ser Leu Asp Lys Ala Arg Arg Leu Leu Trp Pro Ser Lys Lys Lys Tyr Gly Lys Lys Leu Ser Trp Ala Asp Leu Ile Val.

More particularly, DNA sequences from Mycobacterium tuberculosis can be analyzed by Southern blotting and hybridization. The techniques used for the present invention are described in Maniatis et al. (1989). DNA fragments can be separated on agarose gels and denatured in situ. The fragments can then be transferred from the gel to a water insoluble solid, porous support, such as a nitrocellulose filter, a nylon membrane, or an activated cellulose paper, where they are immobilized for example, the Hybond® membrane commercialized by Amersham can be used. After prehybridization to reduce non-specific hybridization with the probe, the solid support is hybridized to the nucleic acid probe of the invention. The solid support is washed to remove unbound and weakly binding probe, and the resulting hybrid duplex molecule is examined. A convenient alternative approach is to hybridize oligonucleotides to the DNA denatured in the gel.

The amount of labeled probe which is present in the hybridization solution will vary widely, depending upon the nature of the label, the amount of the labeled probe which can reasonably bind to the filter, and the stringency of the hybridization. Generally, substantial excesses of the probe over stoichiometric will be employed to enhance the rate of binding of the probe to the fixed DNA.

Various degrees of stringency of hybridization can be employed. The more severe the conditions, the greater the complementarity that is required for hybridization between the probe and the polynucleotide for duplex formation. Severity can be controlled by temperature, probe concentration, probe length, ionic strength, time, and the like. Conveniently, the stringency of hybridization is varied by changing the polarity of the reactant solution. Temperatures to be employed can be empirically determined or determined from well known formulas developed for this purpose.

Unlike Southern hybridization where DNA fragments are transferred from an agarose gel to a solid support, the method of the invention can also be carried out by oligonucleotide hybridization in dried agarose gels. In this procedure, the agarose gel is dried and hybridization is carried out in situ using an oligonucleotide probe of the invention. This procedure is preferred where speed of detection and sensitivity may be desirable. The procedure can be carried out on agarose gels containing genomic or cloned DNA of Mycobacterium tuberculosis.

In addition, the method of this invention can be carried out by transfer of Mycobacterium tuberculosis DNA from polyacrylamide gels to nylon filters by electroblotting. Electroblotting may be desirable where time is of the essence, because electroblotting is typically faster than capillary blotting developed to transfer DNA from agarose gels. This method can be carried out in conjunction with UV-crosslinking. The polyacrylamide gel containing the samples to be tested is placed in contact with an appropriately prepared nylon filter. These are then sandwiched into an electroblotting apparatus and the DNA is transferred from the gel onto the filter using electric current. After a buffer rinse, the filter is ready to be prehybridized and hybridized or UV-crosslinked.

The method of the invention can be carried out using the nucleic acid probe of the invention for detecting Mycobacterium tuberculosis resistant to isoniazid. The probe can be detected using conventional techniques.

The nucleotides of the invention can be used as probes for the detection of a nucleotide sequence in a biological sample of M. tuberculosis. The polynucleotide probe can be labeled with an atom or inorganic radical, most commonly using a radionuclide, but also perhaps with a heavy metal. Radioactive labels include ³²P, ³H, ¹⁴C, or the like. Any radioactive label can be employed, which provides for an adequate signal and has sufficient half-life. Other labels include ligands that can serve as a specific binding member to a labeled antibody, fluorescers, chemiluminescers, enzymes, antibodies which can serve as a specific binding pair member for a labeled ligand, and the like. The choice of the label will be governed by the effect of the label on the rate of hybridization and binding of the probe to the DNA or RNA. It will be necessary that the label provide sufficient sensitivity to detect the amount of DNA or RNA available for hybridization.

In preferred embodiments of the invention, the probe is labeled with a radioactive isotope, e.g., ³²P or ¹²⁵I, which can be incorporated into the probe, e.g., by nick-translation.

In other preferred embodiments, the probe is labeled with biotin, which reacts with avidin to which is bonded a chemical entity which, when the avidin is bonded to the biotin, renders the hybrid DNA complex capable of being detected, e.g., a fluorophore, which renders the hybrid DNA complex detectable fluorometrically; an electron-dense compound capable of rendering the hybrid DNA complexes detectable by an electron microscope; an antibody capable of rendering the hybrid DNA complexes immunologically detectable; or one of a catalyst/substrate pair capable of rendering the hybrid DNA complexes enzymatically detectable. Prior to contacting the bacteria with the probe, the M. tuberculosis bacteria can be lysed to release their DNA, which is then denatured and immobilized on an appropriate solid, DNA-binding support, such as a nitrocellulose membrane.

Another detection method, which does not require the labeling of the probe, is the so-called sandwich hybridization technique. In this assay, an unlabeled probe, contained in a single-stranded vector, hybridizes to isoniazid-sensitive Mycobacterium tuberculosis DNA, and a labeled, single-stranded vector, not containing the probe, hybridizes to the probe-containing vector, labeling the whole hybrid complex.

The sequences of the invention were derived by dideoxy-nucleotide sequencing. The base sequences of the nucleotides are written in the 5'-----> 3' direction. Each of the letters shown is a conventional designation for the following nucleotides:
- A: Adenine
- G: Guanine
- T: Thymine
- C: Cytosine.
The nucleotides of the invention can be prepared by the formation of 3'-----> 5' phosphate linkages between nucleoside units using conventional chemical synthesis techniques. For example, the well-known phosphodiester, phosphotriester, and phosphite triester techniques, as well as known modifications of these approaches, can be employed. Deoxyribonucleotides can be prepared with automatic synthesis machines, such as those based on the phosphoramidite approach. Oligo- and polyribonucleotides can also be obtained with the aid of RNA ligase using conventional techniques.

The nucleotides of the invention are in a purified form. For instance, the nucleotides are free of human blood-derived proteins, human serum proteins, viral proteins, nucleotide sequences encoding these proteins, human tissue, and human tissue components. In addition, it is preferred that the nucleotides are free of other nucleic acids, extraneous proteins and lipids, and adventitious microorganisms, such as bacteria and viruses.

Since it may be possible to increase the sensitivity of detection by using RNA instead of chromosomal DNA as the original template, this invention contemplates using RNA sequences that are complementary to the DNA sequences described herein. The RNA can be converted to complementary DNA with reverse transcriptase and then subjected to DNA amplification.

### EXPERIMENTAL PROCEDURES

### Bacterial strains and plasmids

Table 1 outlines the properties of the bacterial strains and plasmids used in this invention.

**Table 1.**

| **Bacterial Strains And Plasmids** | |
|---|---|
| Strains/plasmids | Characteristics |
| E. coli NM554 | |
| | |
| E. coli TG1 | supE hsd5 thi delta (lac-proAB) [traD36 proAB+ lacI^{g} lacZ delta M15] |
| E. coli UM2 | KatE |
| | |
| E. coli UM255 | KatE |
| | |
| M. tuberculosis H37Rv | Virulent strain originally isolated from tuberculosis patient |
| | |
| M. tuberculosis 12 | Clinical isolate resistant to low levels of INH (1-2 µg/ml) |
| | |
| M. tuberculosis B1453 | Clinical isolate resistant to high levels of INH (>50 µg/ml) |
| | |
| M. tuberculosis 24 | Clinical isolate resistant to high levels of INH (>50 µg/ml) |
| | |
| M. tuberculosis 79112 | Clinical isolate sensitive to INH |
| | |
| M. tuberculosis 12646 | Clinical isolate sensitive to INH |
| | |
| M. tuberculosis 79665 | Clinical isolate sensitive to INH |
| | |
| M. smegmatis MC²155 | MC²6 het |
| | |
| M. smegmatis BH1 | MC²155 het katG |

| Plasmids | |
|---|---|
| pBH4 | Shuttle cosmid, katG+, based on pYUB18 |
| | |
| pBH5 | Deleted version of pBH4, katG+, (7 kb-EcoRI) |
| | |
| pYZ55 | pUC19 derivative with 4.5 kb KpnI fragment, kat+ |
| | |
| pYZ56 | pUC19 derivative with 2.5 kb EcoRV-KpnI fragment (kat+) |
| | |
| pYZ57 | pUC19 derivative with 3.1 kb KpnI-BamHI fragment, kat- |
| | |
| pBAK14 | Mycobacterial shuttle vector (Zhang et al., 1991) |
| | |
| pBAK15 | Mycobacterial shuttle vector carrying 4.5 kb KpnI fragment (kat+) |
| | |
| pBAK16 | Mycobacterial shuttle vector carrying 2.5 kb EcoRV-KpnI fragment (kat⁺) |
| | |
| pBAK17 | Mycobacterial shuttle vector carrying 3.1 kb KpnI-BamHI fragment (kat⁻) |

The M. tuberculosis H37 RV genomic library was constructed in the shuttle cosmid pYUB18 (Snapper et al., 1988) and kindly supplied by Dr. W. R. Jacobs. Other shuttle vectors employed were pYUB12 (Snapper et al., 1988) and pBAK14 (Zhang et al., 1991).

### Microbiological techniques and enzymology

Details of antibiotics used, growth conditions, enzymology and MIC determinations can be found in Heym et al., (1992).

### Nucleic acid techniques

Standard protocols were used for subcloning, Southern blotting, DNA sequencing, oligonucleotide biosynthesis, etc. (Maniatis et al., 1989; Eiglmeier et al., 1991).

### Activity staining

The preparation of cell-free extracts of E. coli and mycobacteria has been described (Heym et al., 1992; Zhang et al., 1991). Native protein samples were separated by polyacrylamide gel electrophoresis as described by Laemmli (1970) except that SDS was omitted from all buffers, samples were not boiled and betamercaptoethanol was not included in the sample buffer. After electrophoresis of 50 - 100 µg protein samples on 7.5% polyacrylamide gels, catalase activity was detected by soaking the gel in 3mM H₂O₂ for 20 minutes with gentle shaking. An equal volume of 2% ferric chloride and 2% potassium ferricyanide was added and clear bands of catalase activity revealed by illumination with light. Peroxidase activity was detected as brown bands after soaking gels in a solution containing 0.2 - 0.5 mg/ml diaminobenzidine and 1.5 mM H₂O₂ for 30 - 120 minutes.

To generate a highly toxic compound it seems most likely that the M. tuberculosis HPI enzyme peroxidatively activates INH (Youatt, 1969; Gayathri-Devi et al., 1975). Now that the katG gene has been isolated and characterized, it should be possible to make new derivatives of INH, which can be activated in a similar manner.

### Example 1 (comparative)

### Point mutations in the katG gene associated with the isoniazid-resistance of M. tuberculosis

It has been shown in a recent study that the catalase-peroxidase of Mycobacterium tuberculosis, encoded by the katG gene, is involved in mediating the toxicity of the potent anti-tuberculosis drug isoniazid or INH. Mutants resistant to clinical levels of INH show reduced catalase-peroxidase activity and, in some cases, this results from the deletion of the katG gene from the chromosome. Transformation of INH-resistant strains of Mycobacterium smegmatis and M. tuberculosis with the cloned katG gene leads to restoration of drug-sensitivity. Expression of katG in some strains of Escherichia coli renders this naturally resistant organism susceptible to high concentrations of INH.

As some INH-resistant clinical isolates of M. tuberculosis have retained an intact katG gene, the molecular basis of their resistance was investigated. This study was facilitated by the availability of the nucleotide sequence of a 4.7 kb KpnI fragment from the katG region of the chromosome as this allowed primers suitable for PCR analysis to be designed. Eleven pairs of oligonucleotide primers were synthesized (see Table 2) and used to generate PCR-products, of around 280 bp, that covered the complete katG gene and some of the flanking sequences. In control experiments all experiments all eleven primer pairs generated PCR products of the expected size, highly suitable for SSCP-analysis, so a panel of 36 INH-resistant strains of M. tuberculosis, of Dutch or French origin, was examined. Many of these strains are multidrug resistant and were isolated from patients who were HIV-seropositive.

**Table 2.Sequence of primer pairs used for PCR-SSCP analysis of the katG gene of M.tubercula is**

| | | 5' | 3' | Length | G+C(%) | Tm | Production |
|---|---|---|---|---|---|---|---|
| Primer Pair # | 1 | | | | | | |
| OLIGO1: | GCGGGGTTATCGCCGATG | 1765 | 1782 | 18 | 66 | 61.8 | 288 |
| OLIG02: | GCCCTCGACGGGGTATTTC | 2052 | 2034 | 19 | 63 | 61.9 | |
| Primer Pair # | 2 | | | | | | |
| OLIGO1: | AACGGCTGTCCCGTCGTG | 2008 | 2025 | 18 | 66 | 61.9 | 300 |
| OLIGO2: | GTCGTGGATGCGGTAGGTG | 2307 | 2289 | 19 | 63 | 61.9 | |
| Primer Pair # | 3 | | | | | | |
| OLIGO1: | TCGACTTGACGCCCTGACG | 2169 | 2187 | 19 | 63 | 61.9 | 280 |
| OLIGO2: | CAGGTCCGCCCATGACAG | 2448 | 2431 | 18 | 66 | 61.9 | |
| Primer Pair # | 4 | | | | | | |
| OLIGO1: | CCACAACGCCAGCTTCGAC | 2364 | 2382 | 19 | 53 | 61.9 | 284 |
| OLIGO2: | GGTTCACGTAGATCAGCCCC | 2647 | 2628 | 20 | 50 | 61.9 | |
| Primer Pair # | 5 | | | | | | |
| OLIGO1: | GCAGATGGGGCTGATCTACG | 2622 | 2641 | 20 | 60 | 51.9 | 288 |
| OLIGO2: | ACCTCGATGCCGCTGGTG | 2909 | 2892 | 18 | 66 | 51.9 | |
| Primer Pair # | 6 | | | | | | |
| OLIGO1: | GCTGGAGCAGATGGGCTTG | 2829 | 2847 | 19 | 63 | 61.9 | 286 |
| OLIGO2: | ATCCACCCGCAGCGAGAG | 3114 | 3097 | 18 | 66 | 61.9 | |
| Primer Pair # | 7 | | | | | | |
| OLIGO1: | GCCACTGACCTCTCGCTG | 3088 | 3105 | 10 | 66 | 61.9 | 297 |
| OLIGO2: | CGCCCATGCGGTCGAAAC | 3384 | 3367 | 18 | 66 | 61.9 | |
| Primer pair # | 8 | | | | | | |
| OLIGO1: | GCGAAGCAGATTGCCAGCC | 3304 | 3322 | 19 | 63 | 61.9 | 285 |
| OLIGO2: | ACAGCCACCGAGCACGAC | 3588 | 3571 | 18 | 66 | 61.9 | |
| Primer Pair # | 9 | | | | | | |
| OLIGO1: | CAAACTGTCCTTCGCCGACC | 3549 | 3568 | 20 | 60 | 61.9 | 281 |
| OLIGO2: | CACCTACCAGCACCGTCATC | 3829 | 3810 | 20 | 60 | 61.9 | |
| Primer Pair # | 10 | | | | | | |
| OLIGO1: | TGCTCGACAACGCGAACCTG | 3770 | 3789 | 20 | 60 | 61.9 | 280 |
| OLIGO2: | TCCGAGTTGGACCCGAAGAC | 4049 | 4030 | 20 | 60 | 61.9 | |
| Primer Pair # | 11 | | | | | | |
| OLIGO1: | TACCAGGGCAAGGATGGCAG | 3973 | 3992 | 20 | 60 | 61.9 | 280 |
| OLIGO2: (#courier10) | GCAAACACCAGCACCCCG | 4252 | 4235 | 18 | 66 | 61.9 | |

Two of them gave no PCR fragment, with any of the primers used, indicating that katG had been deleted. The remaining 34 strains all yielded the expected PCR products and these were analyzed on SSCP gels so that possible point mutations could be detected. In 20 cases, abnormal strand mobility was observed, compared to that of katG from drug-sensitive M. tuberculosis, suggesting that mutational events had indeed occurred. The approximate locations of the mutations, as delimited by the PCR primers, are shown in Table 3.

**Table 3. Preliminary results of PCT-SSCP analysis of katG from M. tuberculosis strains x denotes altered mobility; del denotes deletion**

| N° | Strain | MIC (INH) | 1 | 2 | 3 | 4 | 5 | 6 | 7 |
|---|---|---|---|---|---|---|---|---|---|
| | | | 1765-2034 | 2008-2289 | 2169-2431 | 2364-2628 | 2622-2892 | 2829-3097 | 3088 3367 |
| 1/37 | 9488 | 1 | | | | | | | X |
| 2 | 9577 | 1 | | | | | | | |
| 3 | 9112 | 10 | | | | | | | |
| 4 | 9247 | 1 | | | | | | | |
| 5 | 9200 | 1 | | | | | X | | |
| 6 | 9116 | 1 | | | | | | | |
| 7/31 | 9106 | 1 | | | | | X | | X |
| 8 | 9291 | 1 | | | | | | | X |
| 9/10 | 9412 | 1 | X | | | | | | |
| 11/12 | 9435 | 1 | | | | | | | |
| 13 | 9428 | 1 | | | X | | | | |
| 14 | 9441 | 1 | | | X | | | | X |
| 15/16 | 9444 | 1 | | | X | | | | X |
| 17/18 | 9445 | 1 | | | | | | | |
| 19/20 | 9330 | 0,2 | | | X | | | | |
| 21/22 | 9420 | 0,2 | | | | | | | |
| 23 | 9262 | 0,2 | | | | | | | |
| 24/38 | 9523 | 1 | | | | | X | | |
| 25 | 9592 | 10 | | | X | | | | |
| 26 | 9553 | 10 | | | | | | | |
| 27 | 9485 | 10 | | | | | X | | |
| 28 | 9181 | 1 | | | X | | X | | |
| 29 | 9363 | 1 | | | | | | | |
| 30 | 9465 | 1 | | | X | | | | X |
| 32 | 9178 | 0,2 | | | | | | | |
| 33 | 9468 | 0,2 | | | | | | | |
| 34 | 9218 | 0,2 | | | | | | | |
| 33 | 9468 | 0,2 | | | | | | | |
| 34 | 9218 | 0,2 | | | | | | | |
| 35 | 9503 | 0,2 | | | | | | | |
| 39 | 9582 | 1 | | | | | X | | |
| 41 | H37Rv | - | | | | | | | |
| 42 | Ass | - | | | | | | | |
| 43 | Mou | - | | | | | | | |
| 44 | 13632 | >20 | del | del | del | del | del | del | del |
| 45 | 13549 | >5 | del | del | del | del | del | del | del |
| 46 | 13749 | >20 | | | | | | | |
| 47 | 14006 | 10 | | | | | | | X |
| 48 | 13711 | >5 | | | | | | | |
| 49 | 13681 | >5 | | | | | X | | |
| 50 | 14252 | >5 | | | | | | | |

On examination of a 200 bp segment of the katG gene from five independent strains (9188, 9106, 9441, 9444, 9363), a single base difference was found. This was the same in all cases, a G to T transversion at position 3360, resulting in the substitution of Arg-461 by Leu. Thus, in addition to inactivation of katG, INH-resistance can stem from mis-sense mutations that result in an altered catalase peroxidase. This mutation may define a site of interaction between the drug and the enzyme. The results of DNA sequence studies with the remaining mutants are eagerly awaited.

Another conclusion that can be drawn from this study concerns the molecular basis of the multidrug resistance associated with various M. tuberculosis strains. The same mutations are found irrespective of whether a given patient is seropositive or seronegative for HIV. For example, strain 9291, isolated from an HIV-seropositive tuberculosis patient, harbors mutations conferring resistance to INH, rifampin and streptomycin in the katG (R461L), rpoB (S425L) and rpsL (K42R) genes, respectively. The same mutations have been found separately, or in combination, in strains from HIV-seronegative individuals. This means that, for the set of strains studied, there is no novel, single mechanism conferring resistance to several drugs, but rather, multidrug resistance results from the accumulation of mutations in the genes for distinct drug targets.

### Example 2

### Nucleotide sequence and chromosomal location of the katG locus of M. tuberculosis

**Bacterial strains, plasmids and growth conditions.** The following bacterial strains from our laboratory collections were used in this study: M. tuberculosis H37Rv; M. smegmatis MC²155 (Snapper et al., 1990); E. coli K-12 UM2 (katE katG; Mulvey et al., 1988). The recombinant plasmids, pYZ55 (pUC19, katG⁺), pYZ56 (pUC19, lacZ'::katG) and the shuttle clones, pBH4 (pYUB18, katG⁺) and pBAK-KK- (pBAK14, katG⁺) have been described recently (Zhang et al. 1992, Nature) and the katG locus of M. tuberculosis is schematized in Fig. 5. Mycobacteria were grown at 37°C in Middlebrook 7H9 medium, while E. coli strains were cultivated in L-broth, with appropriate enrichments and antibiotics.

**Nucleic acid techniques.** Standard techniques were employed for the preparation, labelling and hybridization of DNA (Eiglmeier et al. 1991; Zhang et al. 1992, Infect. Immun.; Zhang et al. 1992, Nature). A shotgun library of random fragments of pYZ55 was prepared in M13mp18 as described previously (Garnier et al., 1986) and sequenced using the modified dideoxy technique (Biggin et al. 1983). Sequences were compiled and assembled into contigs using SAP, and analyzed with NIP, SIP and PIP (Staden 1987) running on a Vax 3100 workstation. Gap closure was obtained by using synthetic oligonucleotide primers, synthesized on an ABI 381 apparatus, and T7 DNA polymerase (Pharmacia) to obtain sequences directly from pYZ55. To search for related sequences in the GenBank database (release 73.1) the FASTA (Pearson et al. 1988) and BLAST (Altschul et al. 1990) programs were used. The PROSITE (Bairoch 1992) catalog was screened to detect possible motifs present in protein sequences and alignments were done with the PILEUP and PRETTY modules of the GCG sequence analysis package (Devereux et al. 1984).

**Western blotting and catalase-peroxidase activity staining**. Immunoblotting of polypeptides resolved by SDS-polyacrylamide gel electrophoresis and detection with polyclonal antibodies (purchased from DAKO) raised against M. bovis BCG, were as described (Zhang et al. 1992, Infect. Immun., Nature, Mol. Microbiol.). Procedures for detecting catalase and peroxidase activities have been outlined recently (Heym et al. 1992; Zhang et al. 1992, Nature).

### RESULTS

**Nucleotide sequence of the katG locus of M. tuberculosis**. In previous studies, the complete katG gene was cloned independently in E. coli on a shuttle cosmid, pBH4, and on a 4.5 kb KpnI restriction fragment thus giving rise to pYZ55 (Fig. 5; Zhang et al. 1992, Nature). The structural gene for catalase-peroxidase was subsequently localized to a 2.5 kb EcoRV - KpnI fragment by sub-cloning. To deduce the primary structure of this important enzyme and thereby gain some insight into its putative role in the conversion of INH into a potent anti-tuberculous derivative, the nucleotide sequence of the complete insert from pYZ55 was determined. This was achieved by the modified dideoxy-shotgun cloning procedure (Biggin et al. 1993) and gaps between the contigs were closed by using specific primers.

On inspection of the resultant sequence which is shown in Fig. 6A, the 4.5 kb fragment was found to contain 4795 nucleotides with an overall dG+dC content of 64.4%. When this was analyzed for the presence of open reading frames, with high coding-probability values, a single candidate was detected and, from its size, composition and location, this was identified as katG. The absence of any additional open reading frames, on either strand of the KpnI fragment, ruled out the possibility that genes other than katG were involved in conferring INH-susceptibility.

Further analysis of the sequence showed katG to be preceded by two copies of a 700 bp direct repeat which were 68% identical, with the longest stretch of identity comprising 58 bp (Fig. 6B). When the databases were screened with this sequence no significant homologies were detected. To test the possibility that it could correspond to a new repetitive element in M. tuberculosis, a 336 bp probe, encompassing the 58 bp repeat, was used to probe a partially-ordered cosmid library. Positive hybridization signals were only obtained from clones that were known to carry katG. Likewise, a single restriction fragment was detected in Southern blots of M. tuberculosis DNA digested with restriction enzymes BamHI, KpnI and RsrII thereby indicating that this repetitive sequence is not dispersed.

**Chromosomal location of katG.** As part of the M. tuberculosis genome project, most of the genes for which probes are available have been positioned on the contig map. From the series of overlapping cosmids shown in Fig. 5 it can be seen that the markers linked to katG are LL105 and fbpB encoding an anonymous antigen and the putative fibronectin binding protein, or alpha antigen (Matsuo et al. 1988), respectively. None of the known insertion sequences IS6110 and IS1081 (Collins et al. 1991; McAdam et al. 1990; Thierry et al. 1990, J. Clin. Microbiol.; Thierry et al. 1990, Nucleic Acids Res.), map to this area of the chromosome although the region upstream of katG is densely populated with copies of the major polymorphic tandem repeat, MPTR (Hermans et al. 1992; Zhang and Young 1993).

**Presence of katG homologues in other mycobacteria**. INH is exquisitely potent against members of the tuberculosis complex yet shows little, if any, activity against other mycobacteria. To determine whether genes homologous to katG were present in other mycobacteria Southern blots of DNA digested with RsrII were hybridized with a probe prepared from a 2.5 kb EcoRV-KpnI restriction fragment carrying katG from M. tuberculosis. Under conditions of high stringency good signals were obtained from M. leprae and M. avium (Fig. 7) while barely discernible hybridization was observed with M. gordonae and M. szulgai. It has been shown recently that katG homologues are also present in M. Smeamatis and M. aurum (Heym et al. 1992).

**Predicted properties of catalase-peroxidase from M. tuberculosis**. The primary structure of catalase-peroxidase, deduced from the nucleotide sequence of katG, is shown in Fig. 6. The enzyme is predicted to contain 735 amino acids, and to display a molecular weight of 80,029 daltons. A protein of this size has been observed in M. tuberculosis, and both recombinant M. smegmatis and E. coli (see below).

Primary structures are available for several other bacterial catalase-peroxidases including those from E. coli. salmonella typhimurium and Bacillus stearothermophilus (Loewen et al. 1990; Loprasert et al. 1988; Triggs-Raine et al. 1988) and these have been shown to be distantly related to yeast cytochrome c peroxidase (Welinder 1991). As the crystal structure of the latter has been determined (Finzel et al. 1984) this can be used to interpret the sequences of the bacterial enzymes. The M. tuberculosis enzyme shows 53.3% conservation with the enterobacterial HPI enzymes, and shares 45.7% identity with the protein from B. stearothermophilus. An alignment of the sequences of these four enzymes is shown in Fig. 8, along with that of yeast cytochrome c peroxidase (Welinder 1991). It is apparent that the NH₂ terminus, which has no counterpart in the yeast enzyme, is the most divergent part suggesting that this domain of the protein can tolerate extensive deviation and is not required for catalysis. Experimental support for this interpretation is provided in the form of a LacZ-KatG fusion protein which contains an additional 40 amino acid residues (Fig. 9, lane 6; Zhang et al. 1992, Nature). Addition of this NH₂-terminal segment does not noticeably interfere with either the catalase or peroxidase reactions effected by KatG as judged by activity staining (Zhang et al. 1992, Nature).

Bacterial catalase-peroxidases are believed to have evolved by means of a gene duplication event and consist of two modules, both showing homology to the yeast enzyme, fused to a unique NH₂-terminal sequence of about 50 amino acid residues (Welinder 1991). The M. tuberculosis enzyme conforms to this pattern and when searched for internal homology using SIP (Staden 1987) it was clear that the region between residues 55-422 was related to the carboxy terminal domain, consisting of amino acids 423-735. Only one of the two active site motifs typical of peroxidases, present in the PROSITE catalog (Bairoch 1992) was found when the M. tuberculosis catalase-peroxidase primary structure was screened as there are two deviations from the consensus around His²⁶⁹ where the second motif should be. (Consensus pattern for peroxidase 1: [DET]-[LIVMT]-x(2) -[LIVM]-[LIVMSTAG]-[SAG]-[LIVMSTAG]-H-[STA]-[LIVMFY]; consensus pattern for peroxidase 2: [SGAT]-x(3)-[LIVMA] -R-[LIVMA]-x-[FW]-H-x-[SAC]; (Bairoch 1992). In addition, a possible ATP-binding motif (G-x-x-x-x-G-K-T) was detected (Balroch 1992) but as this partially overlaps the active site its presence may be purely fortuitous (Fig. 8).

By analogy with yeast cytochrome c peroxidase (Welinder 1991), it was possible to predict a number of structurally and catalytically important residues all of which are located in the NH₂-terminal repeat. His²⁶⁹ should serve as the fifth ligand of the heme-iron while Asp³⁸⁰ should be its hydrogen-bonded partner. Other residues predicted to be involved in active site modulation and H₂O₂ binding are Arg¹⁰⁴, Trp¹⁰⁷, His¹⁰⁸, Asn¹³⁸, Th²⁷⁴, and His²⁷⁵ (Fig. 4). According to Welinder's predictions (Welinder 1991), Trp³²⁰ should be a key residue and be required for forming the protein-radical site (Sivaraja et al. 1989).

**Antibody response to M. tuberculosis** KatG. To evaluate the possible value of KatG as an immunogen, Western blots were probed with anti-serum raised against M. bovis BCG in rabbits. As shown in Fig. 9, the 80 kD catalase-peroxidase is one of the prominent antigens recognized in cell-free extracts of M. tuberculosis, and M. smegmatis expressing the cloned katG gene (lanes 1, 3). Likewise, on introduction of the gene into E. coli significant levels of catalase-peroxidase were produced a striking increase in expression was obtained from the lacZ'-katG gene fusion which directed the synthesis of an 85 kD fusion protein (Fig. 9, lane 6).

The aim of the present study was to determine the nucleotide sequence of the katG gene and to use the information obtained to try and understand how its product mediates the INH-susceptibility of M. tuberculosis and, possibly, to explain the apparent instability of the katG region of the genome. Repetitive DNA is often a source of chromosomal rearrangements and analysis of the DNA sequence upstream of katG revealed two copies of a 700 bp direct repeat. Since this element appears to be confined to this locus it is unlikely to serve as a target for an event, such as homologous recombination, which could lead to the deletion of the gene that is observed so frequently (Zhang et al. 1992, Nature; Zhang and Young 1993). Likewise, as a 70 kb stretch of the chromosome of M. tuberculosis H37Rv, encompassing katG, is devoid of copies of IS6110 and IS1081, these insertion sequences do not appear to be likely sources of instability. Rather, the presence of a cluster of major polymorphic tandem repeats, MPTR (Fig. 5; Hermans et al. 1992) situated upstream of katG, suggests that this might act as a recombinational hotspot. It may remove both the MPTR cluster and katG (Zhang and Young 1993). The availability of the sequence of the katG region will allow primers suitable for the polymerase chain reaction to be designed and thus facilitate studies aimed at both rapid detection of INH-resistance and understanding the molecular basis of chromosomal instability.

Perhaps the most intriguing feature of the M. tuberculosis catalase-peroxidase is its ability to mediate INH-susceptibility. In our current working hypothesis, the drug interacts with the enzyme and is converted by the peroxidase activity into a toxic derivative which acts at a second, as yet unknown, site (Zhang et al. 1992, Nature). Although horse radish peroxidase can effect this reaction (Pearson et al. 1988; Shoeb et al. 1985), and produce hydroxyl and organic free radicals, very few bacteria, including other mycobacteria, are sensitive to INH. This is intriguing as they contain genes homologous to katG (Fig. 7). One explanation for this could be provided by the fact that most bacterial contain two catalases, one of which is a broad spectrum enzyme endowed with peroxidase activity, and that the second catalase, by preferentially removing H₂O₂, limits the ability of the catalase-peroxidase to oxidize INH. As M. tuberculosis lacks the latter activity its KatG enzyme can convert INH to the lethal form without competition for the electron acceptor.

Alternatively, there may be some unique features of the M. tuberculosis enzyme which promote toxicity or favor the interaction with the drug. Examination of the primary structures of the bacterial catalase-peroxidases was not instructive in this respect as they all share extensive sequence identities and contain two motifs characteristic of the active sites of peroxidases. Furthermore, it has been shown recently that expression of the E. coli katG gene can partially restore INH-susceptibility to drug-resistant mutants of M. tuberculosis suggesting that the endogenous enzyme may not possess any drug-specific properties (Zhang et al. 1993). Sequence comparison with the cytochrome c peroxidase from yeast has provided important information about the structural and functional organization of the KatG protein and led to the identification of the putatively-important catalytic residues (Fig. 8).

Now that the complete sequence of katG is available it will be possible to test some of these hypotheses by site-directed mutagenesis and to overproduce the enzyme so that detailed analysis of the enzymatic reaction, and its products, can be performed in vitro. Likewise, it should be a relatively simple matter to isolate mutants that have retained enzymatic activity but are unable to bind or oxidize INH. Of particular interest is the repetitive structure of the enzyme and the prediction that the NH₂-terminal repeat contains the active site for peroxidases. This raises the possibility that katG genes, mutated, or truncated at the 3'-end, could arise. It is conceivable that their products, lacking the normal COOH-terminus which may be required for subunit-subunit interactions (Welinder 1991), would be unstable but still retain low enzyme activity. They would thus confer an intermediate level of INH-susceptibility, between that of katG⁺ strains and mutants completely lacking the gene, as is often observed in clinical settings.

The invention also relates to a kit for detecting multidrug resistant variants of M. tuberculosis wherein the kit comprises:
(a) a container means containing a probe for the gene encoding drug resistance; and
(b) a container means containing a control preparation of nucleic acid.

A preferred process alternative (oligotyping) for the detection of resistance to the selected antibiotic comprises:
- fragmenting the relevant gene or part thereof likely to carry the mutation into a plurality of fragments, such as by digestion of said relevant gene by selected restriction enzymes,
- hybridizing these fragments to complementary oligonucleotide probes, preferably a series of labelled probes recognizing under stringent conditions, all of the parts of the relevant gene of a corresponding control DNA of a strain non-resistant to the corresponding antibiotic,
- and relating the absence of hybridization of at least one of said oligonucleotide probes to any of the DNA fragments of the relevant gene of the mycobacterium under study as evidence of the presence of a mutation and, possibly, of a resistance to the corresponding antibiotic, particularly as compared to results obtained upon running of the test under the same conditions with the same oligonucleotides on the relevant gene(s) obtained from a strain (strains) not resistant to said antibiotic, wherein said relevant gene is either the rpoB gene or a fragment thereof which gene or fragment thereof is capable of hybridising with the sequence shown in Figure 13, or the rpsL gene, or a fragment threof, which gene or fragment thereof is capable of hybridising with the Mycobacterium tuberculosis sequence shown in Figure 14.

Another process alternative (SSCP analysis, i.e. analysis of Single Stranded Conformation Polymorphisms) comprises:
- digesting the DNA to be analyzed, particularly of the relevant gene,
- amplifying the fragments obtained, e.g. by PCR,
- recovering the amplified fragments, and
- separating them from one another according to sizes, e.g. by causing them to migrate, for instance on an electrophoretic gel,
- comparing the sizes of the different fragments with those obtained from the DNA(s) of one or several control strains not resistant to the antibiotic, which had been subjected to a similar assay, and
- relating the polymorphism possibly detected to the existence of a mutation in the relevant gene, accordingly to a possible resistance to the corresponding antibiotic of the strain from which the DNA under study had been obtained, wherein said relevant gene is either the rpoB gene or a fragment thereof which gene or fragment thereof is capable of hybridising with the sequence shown in Figure 13, or the rpsL gene, or a fragment threof, which gene or fragment thereof is capable of hybridising with the Mycobacterium tuberculosis sequence shown in Figure 14.

Needless to say that any other method, including classical sequencing techniques, can resorted to for the achievement of the same purpose.

This method includes that known under the expression "oligotyping" for the detection of polymorphisms, reference is advantageously made to the method disclosed by Orita et al. (reference was already made thereto herebefore) for the detection of polymorphisms based on the conformation of single strands.

In the case of resistance to rifampicin, the relevant gene happens to be the rpoB gene which codes for the βsub-unit of the RNA polymerases of said mycobacteria, or when only part of that gene is being used, preferably that part which includes the codons 400 to 450 of that rpoB gene.

Finally, in the case of resistance to steptomycin, the relevant gene contemplated is that of the rpsL gene that codes for the S12 protein of the small ribosome sub-unit or, when only part of said fragment is being used, preferably that part which includes the codon at the 43 position.

A preferred procedure, particularly in relation to the process alternative making use of PCR amplification is disclosed hereafter.

DNA is obtained from a biological sample (e.g. blood or sputum) after removal of the cellular debris and lysis of the bacterial cells with an appropriate lysis buffer. PCR amplication can be carried out by classical methods, using a pair of primers, whose sequences are respectively complementary to fragments of each of the strands of the DNA to be amplified.

The nucleotide sequences of the present invention can be employed in a DNA amplification process known as the polymerase chain reaction (PCR). See e.g., Kwok et al. (1987). PCR is advantageous because this technique is rapid.

DNA primer pairs of known sequence positioned 10-300 base pairs apart that are complementary to the plus and minus strands of the DNA to be amplified can be prepared by well known techniques for the synthesis of oligonucleotides. One end of each primer can be extended and modified to create restriction endonuclease sites when the primer is annealed to the PBMC DNA. The PCR reaction mixture can contain the PBMC DNA, the DNA primer pairs, four deoxyribonucleoside triphosphates, MgCl₂, DNA polymerase, and conventional buffers. The DNA can be amplified for a number of cycles. It is generally possible to increase the sensitivity of detection by using a multiplicity of cycles, each cycle consisting of a short period of denaturation of the PBMC DNA at an elevated temperature, cooling of the reaction mixture, and polymerization with the DNA polymerase.

Amplified sequences can be detected by the use of a technique termed oligomer restriction (OR). Single-strand conformation polymorphism (SSCP) analysis can be used to detect DNA polymorphisms and point mutations in a variety of positions in DNA fragments. See, Saiki et al. (1985); Orita et al. (1989). For example, after amplification, a portion of the PCR reaction mixture can be separated and subjected to hybridization with an end-labeled nucleotide probe, such as a ³²p labelled adenosine triphosphate end-labeled probe. In OR, an end-labeled oligonucleotide probe hybridizes in solution to a region of the amplified sequence and, in the process, reconstitutes a specific endonuclease site.

The procedure may be run further as follows:
- the amplification products (comprising e.g. from 100 to 300 nucleotides) are digested by means of suitable restriction endonuclease,
- the ADN strands obtained from the amplification medium are subjected to denaturation,
- the monostranded DNA strands are deposited on a neutral 5% polyacrylamide gel,
- the monostranded DNA strands are caused to migrate on said gel by means of electrophoresis,
- the DNA fragments that migrated on the polyacrylamide gel are transferred onto a nylon membrane according to a usual electrophoretic blotting technique and hybridized to labelled probes, for instance ³²P labelled probes, and
- the migration distances of the DNA fragments subjected to analysis are compared to those obtained from controls obtained under the same conditions of amplification, digestion, denaturation electrophoresis and transfer onto a nylon membrane, whereby said DNA had been obtained from an identical bacterial strain yet sensitive to the antibiotic under study.

For the production of the PCR primers as well as of the polygonucleotides probes used in the above disclosed "oligotyping" procedures, use is advantageously made of those complementary to the rpoB gene of wild M.tuberculosis inserted in a plasmid deposited under number I-12167 at the CNCM on September 15, 1992.

The invention also relates more particularly to the nucleotidic sequence of a fragment of rpsL gene of Mycobacterium tuberculosis coding for the S12 protein of the small ribosome sub-unit, as well as to the nucleotidic sequence of a mutated rpsL gene fragment deemed responsible of the resistance to streptomycin.

By amplification of that nucleotidic sequence, the nucleotide sequence of the full rpsL gene can be obtained.

Further illustration of the invention will be provided in the following description of additional examples, having regard to the drawings in which:

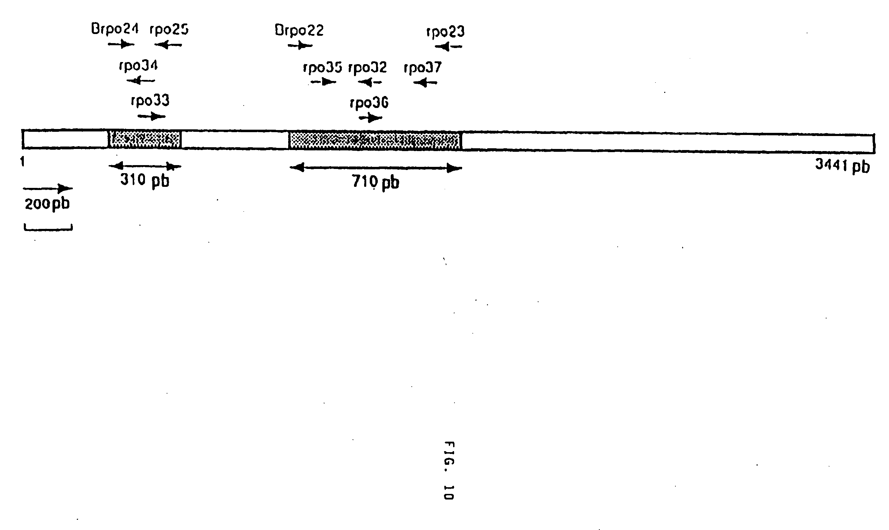

- **figure 10** represents digrammatically the PCR strategy used for the study of different M.Leprae isolates, showing the coding sequence of rpoB sequence, whereby the sequenced regions are shown by hatched parts, and the position and reference of the amplification primers used being indicated on the upper line, whereas the sequencing primers are indicated bellow it;

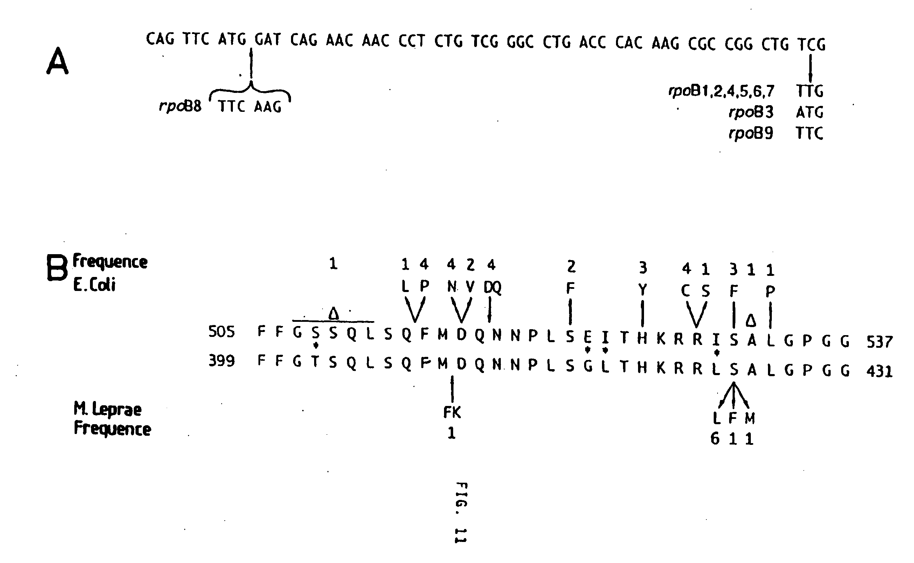

- **figure 11** represents (A) the nucleotidic sequence of a short region of rpoB carrying the mutations that confer resistance to rifampicin with an indication of the changes of bases in the corresponding alleles and (B) a comparison between the aminoacids sequences of the domain I of region II of the β-sub-unit of the RNA polymerase of E. coli and M.Leprae, whereby the numbers of the residues and the differences in the mutated aminoacids have been indicated; the mutated aminoacid residues associated with rifampicin resistance as well as the frequency of its occurrences have been represented too,

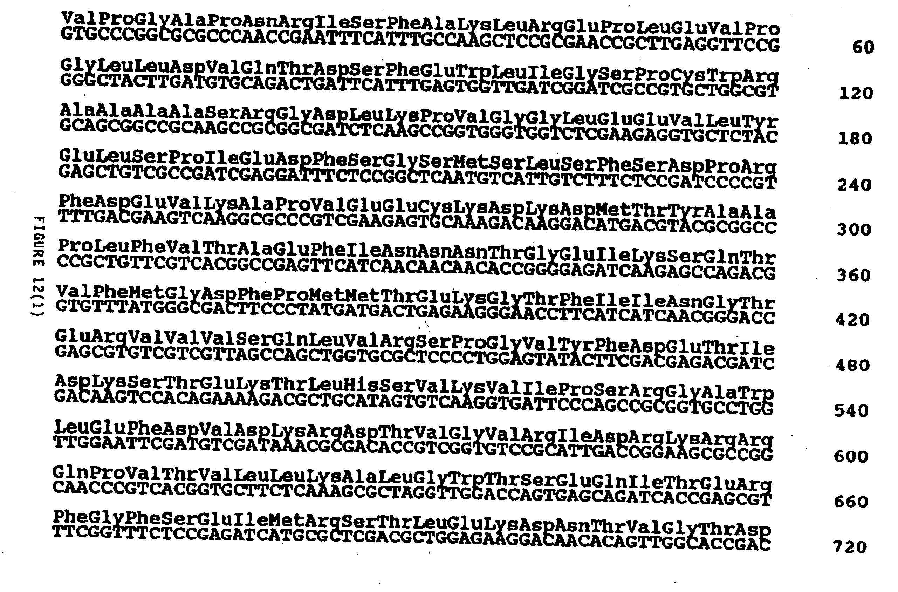

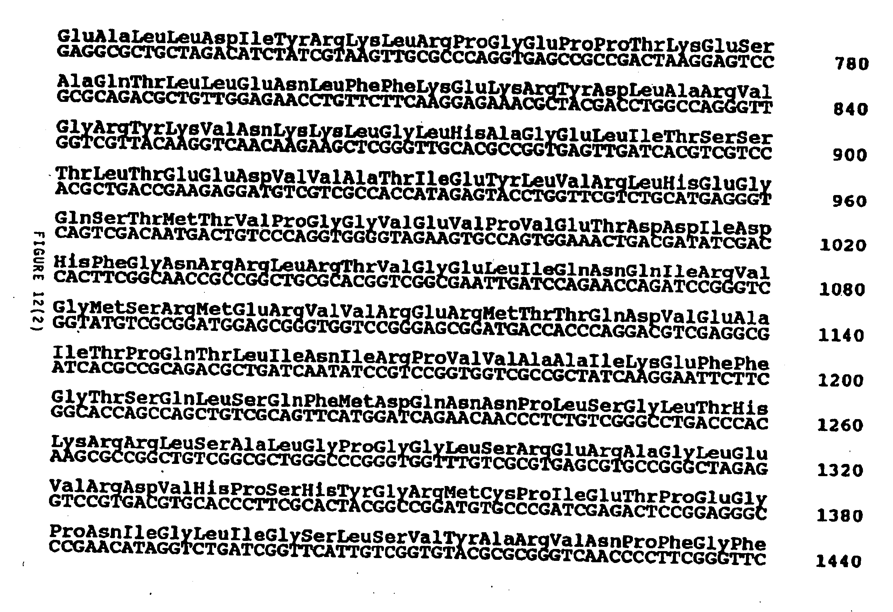

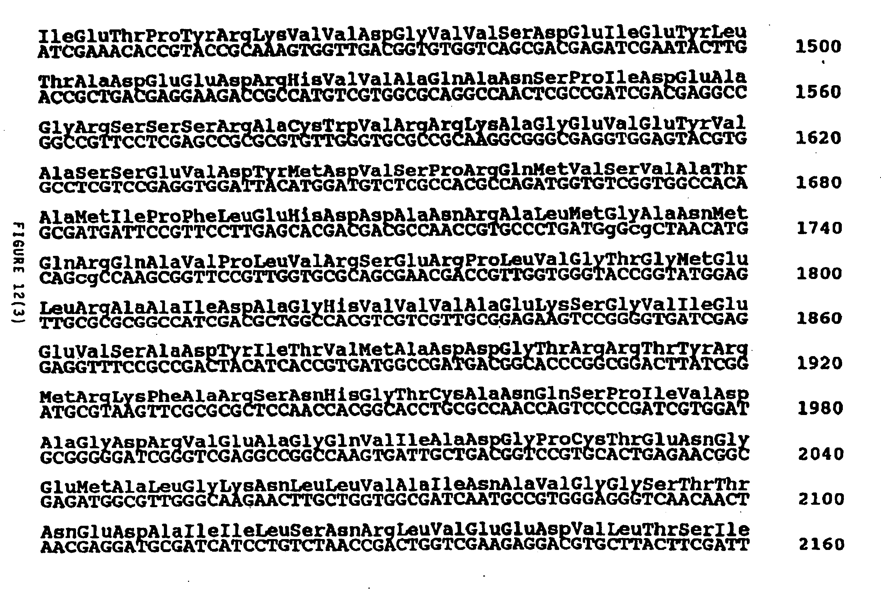

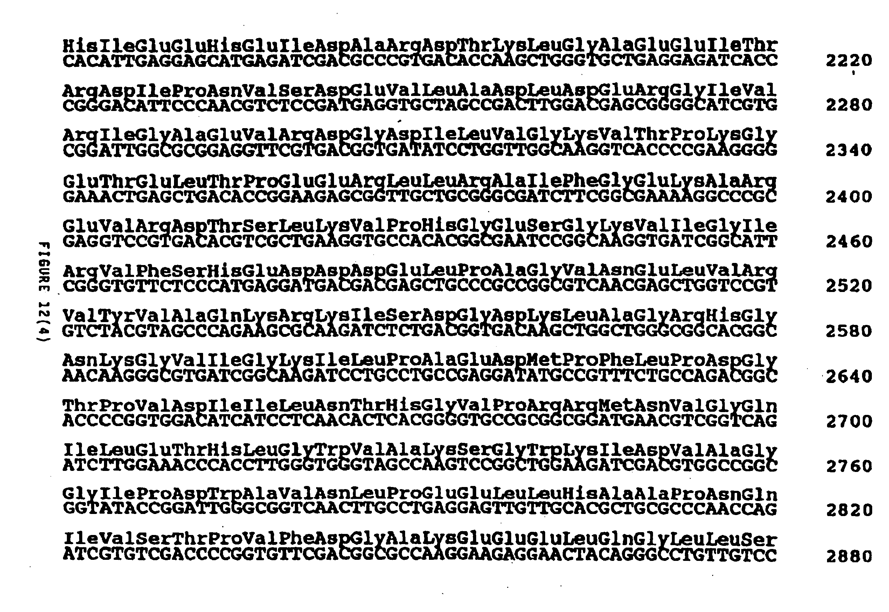

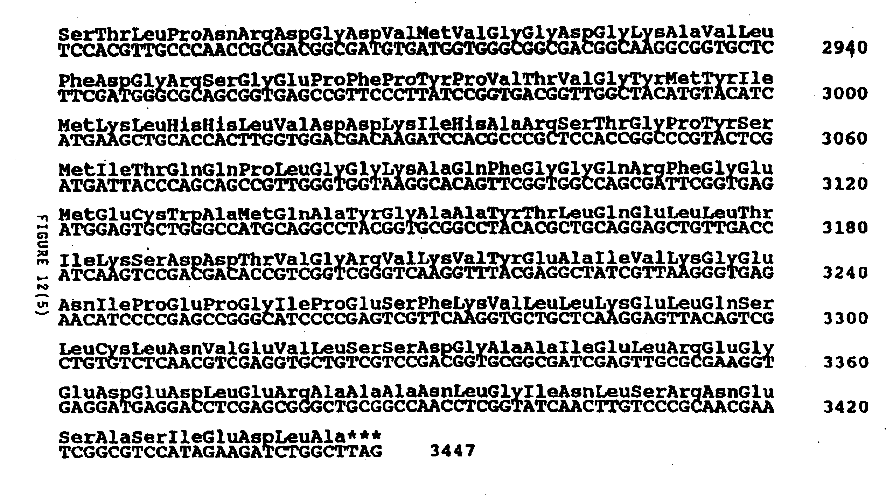

- **figure 12** shows a complete sequence of the rpoB gene of M. Leprae,
- **figure 13** represents the sequence of part of the rpoB gene of M.tuberculosis,
- **figure 14** represents the sequence of a part of the rpsL gene of M.tuberculosis; both the sequence of the full rpsL gene of M.Leprae and that of its expression product, that is the S12 protein (whose starting aminoacid is noted by 1) are indicated. The positions of the ML51 and ML52 primers, as well as of the sequences of part of the rpsL gene of M.tuberculosis are provided belows those of M.Leprae. Only those positions which are different and the corresponding aminoacid changes are indicated.

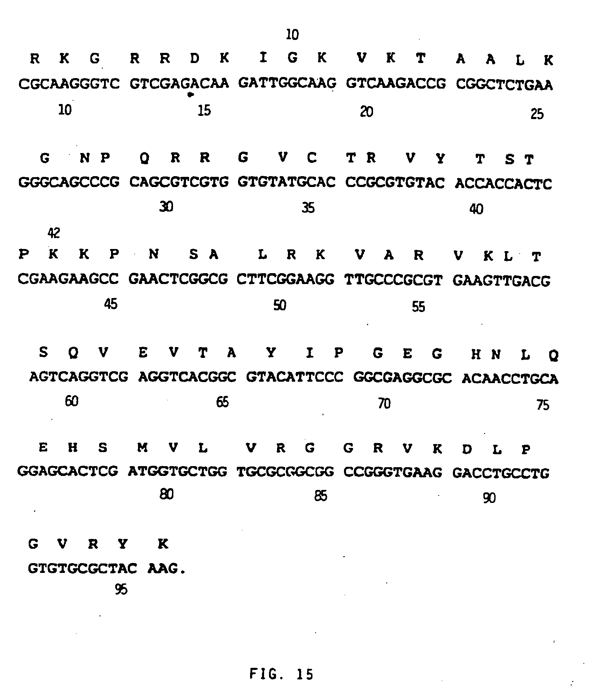

- **figure 15** represents the wild DNA sequence of the rpsL gene fragment coding for the S12 protein of the small ribosome sub-unit that is responsible for the resistance to streptomycin, as well as the corresponding aminoacid sequence of the S12 protein.

### Example relative to the detection of the resistance of Mycobactaria of rifampicin

The sensitivity to rifampicin has been determined in mice as disclosed by Grosset et al. (and Int. J. Lepr. 57:607-614). The cells of M.Leprae were obtained from mouse paws according to classic procedures. All resistant strains were able to grow in mice which received daily doses of 20 mg/Kg of rifampicin, whereas sensitive strains were killed at low rifampicin concentrations, less than 2 mg/Kg.

Relevant regions of the rpoB gene of extracted DNA was initiated upon using two pairs of biotinylated primers, whose sequences appear in the following table.

**TABLE**

| Primer | Séquence |
|---|---|
| Brpo22 | CAGGACGTCGAGGCGATCAC |
| rpo23 | AACGACGACGTGGCCAGCGT |
| Brpo24 | CAGACGGTGTTTATGGGCGA |
| rpo25 | TCGGAGAAACCGAAACGCTC |
| rpo32 | TCCTCGTCAGCGGTCAAGTA |
| rpo33 | CTTCCCTATGATGACTG |
| rpo34 | GGTGATCTGCTCACTGG |
| rpo35 | GCCGCAGACGCTGATCA |
| rpo36 | TTGACCGCTGACGAGGA |
| rpo37 | GCCAGCGTCGATGGCCG |

Upon using conventional techniques, amplification products comprising 310 and 710 bp were respectively obtained as shown in figure 1. The localization of the sequences of the different primers used in the table is also indicated on figure 10.

The DNAs obtained have been sequenced on the basis of the rpoB sequence of isolates sensitive to rifampicin. A plasmid containing the sequence of that gene has been deposited at CNCM on September 15, 1992 under number I-1266. Biotinylated PCR products were concentrated from the PCR reaction mixtures by contacting with streptavidin coated beads under agitation. The biotinylated strands attached to the beads were then recovered and sequenced. The sequences obtained were compared to the sequence of the rpoB gene of a wild type stain. Significant results were obtained as a result of sequencing of the wild gene (of a mycobacterium sensitive to rifampicin) and of corresponding sequences of the β-sub-unit of four mutant strains resistant to rifampicin (**figure 11**).

Results were obtained starting from 102 strands obtained from patients infected with M.tuberculosis. Among this 102 strands 53 were sensitive to rifampicin and 49 resistant to rifampicin. The mutation was localized in the region 400-450 in 43 of the mutants and among the latter, the mutation occured in the region of ⁴²⁵Ser into leu.

### Example of detection of the resistance of mycobacteria to streptomycin

The culture of M.tuberbulosis strains and the test of their sensitivity to streptomycin have been carried out by the method of proportions on a Löwenstein-Ierva medium (Laboratory Method for Clinical Mycobacteriology - Hugo David - Véronique Lévy Frébault, M.F. Thorel, published by Institut Pasteur).

The nucleotide sequence of the rpsL gene of M.Leprae led, by sequence analogy, to the construction of two primers, ML51 (CCCACCATTCAGCAGCTGGT) et ML52 (GTCGAGCGAACCGCGAATGA) surounding regions including putative mutation sites liable of being responsible for the streptomycin resistance and suitable for the PCR reaction. The DNA of the used M.tuberculosis used as a matrix has enabled one to obtain a rpsL fragment of 306 pb. The nucleotide sequence of the sequenced fragments exhibited 28 differences with that of M. Leprae.

The rpsL genes of 43 strains of M.tuberculosis, among which 28 were resistant, have been amplified both by PCR and the SSCP technique.

DNA was extracted from 200 µl aliquots of M.tuberculosis samples (in average 10⁴ to 10⁵ bacteria) covered by 100µl of mineral oil by a Freeze-thawing technique (Woods and Cole, 1989 FEBS. Microbiol. Lett,65:305-308).

After electrophoresis of the DNA strands tested a mutation was shown in 16 of the mutants. In order to establish the nature of the mutation in the 16 strands under consideration, the corresponding rpsL gene fragments were amplified by PCR using the ML51 and the ML52 primers and their respective nucleotide sequences were determined.

The sequences obtained were compared to the sequence of the wild type rpsL gene. The single difference was found with the wild sequence ; codon 43, AAG, was mutated into AGG and, consequently, the lys-42 aminoacid was replaced by Arg.

The invention further relates to kits for the resistance of mycobacteria to isoniazid, rifampicin or analogues thereof, and streptomycin.

The invention further relates to a kit for the in vitro diagnostic of the resistance of a bacteria of a mycobacterium genus to isoniazid, characterized in that it comprises: - means for carrying out for a genic amplification of the DNA of the katG gene or of a fragment thereof, which gene or fragment thereof is capable of hybridising with a 25 kb Ecor-KnpI fragment of plasmid pYZ56,
- means to bring into evidence one or several mutations on the amplification products so obtained,
- a preparation of control DNA of a katG gene of a strain of said bacteria sensitive to isoniazid or of a fragment thereof,
- optionally, a control preparation of a DNA of the katG gene of an isoniazid-resistant mycobacterium strain.

The invention further relates to a kit for the in vitro diagnostic of the resistance of a bacteria of a mycobacterium genus to rifampicin or its analogues, characterized in that it comprises:
- means for carrying out for a genic amplification of the DNA of the rpoB gene or of the β-sub-unit of the RNA polymerase of said mycobacteria, or of a fragment thereof, which gene or fragment thereof is capable of hybridising with the sequence shown in Figure 13,
- means to bring into evidence one or several mutations on the amplification products so obtained,
- a preparation of control DNA of a rpoB gene coding for the β-sub-unit of the RNA polymerase of a strain of said bacteria sensitive to rifampicin or of a fragment thereof,
- optionally, a control preparation of a DNA of the rpoB gene of an isoniazid-resistant mycobacterium strain.

Similarly, the invention pertains to a kit for the in vitro diagnostics of the resistance of the M.tuberculosis to streptomycin, characterized in that it includes:
- means for carrying out a genic amplification of the spsL gene coding for the S12 protein of the small ribosome subunit, or fragment thereof, which gene or fragment thereof is capable of hybridising with the Mycobacterium tuberculosis sequence shown in Figure 14,
- means which enable the bringing to evidence of one or several mutations on the amplification products obtained,
- a control preparation of a DNA sequence of the rpsL gene coding for the S12 protein of the small sub-unit of the ribosome of a M.tuberculosis strain sensitive to streptomycin, and
- optionally, a control preparation of a DNA sequence of a rpsL gene coding for the S12 protein of the small sub-unit of the ribosome of a strain of M.tuberculosis resis tant to streptomycin.

The invention further relates to a nucleic acid sequence comprising a 2.5 kb EcoRV-KnpI fragment of plasmid pYZ56.

The invention also relates to a nucleic acid sequence comprising a 4.5 kb KnpI fragment of plasmid pYZ55, wherein said fragment contains a BamHI cleavage site.

The invention is also directed to a nucleotide sequence which is the 3447 base sequence as described in Figure 12, or a portion thereof, said portion being :
- the sequence illustrated in Figure 11A ;
- the 710 base pair fragment obtainable by amplification of the sequence illustrated in Figure 12 with the primers CAGGACGTCGAGGCGATCAC and AACGACGACGTGGCCAGCGT ;
- the nucleotide sequence extending from nucleotides 1195 to 1293 in Figure 12, encoding the amino acid sequence illustrated in Figure 11B.

The invention is also directed to a nucleotide sequence which is the 432 base sequence as described in Figure 13, or a portion thereof, said portion being :
- a sequence including the codons 400 - 450 of the rpoB gene ;
- the nucleotide sequence extending from nucleotides 169 to 267 in Figure 13, encoding the amino acid sequence illustrated in Figure 11B.

The invention also concerns the use of a nucleic acid sequence comprising the sequence of any of claims 21 to 28 for the detection of antibiotic resistance in Mycobacteria.

### REFERENCES CITED IN THE SPECIFICATION

Altschul, S., Gish, W., Miller, W., Myers, E., and Lipman, D. (1990). A basic local alignment search tool. Proc. Natl. Acad. Sci. USA 215:403-410.

Bekierkunst, A. & Bricker, A. (1967). Studies on the mode of action of isoniazid on mycobacteria. Arch. Biochem. Biophys. 122:385-392.

Biggin, M.D., Gibson T.J., and Hong G.F. (1983). Buffer gradient gels and 35S-label as an aid to rapid DNA sequence determination. Proc. Natl. Acad. Sci. USA 80:3963-3965.

Bairoch, A., (1992). Prosite: a dictionary of sites and patterns in proteins. Nucleic Acids Res. 20:2013-2018.

C.D.C. Outbreak of multidrug-resistant tuberculosis - Texas, California, and Pennsylvania. MMWR 1990, 39:369-37.2.

C.D.C. Nosocomial transmission of multidrug-resistant tuberculosis among HIV-infected persons - Florida and New York 1988-1991. MMWR 1991(a) 40:585-591.

C.D.C. Transmission of multidrug-resistant tuberculosis from an HIV-positive client in a residential substance abuse treatment facility. Michigan. MMWR 1991(b), 40:129-131.

Chaisson, R.E., Schecter, G.F., Theuer, C.P., Rutherford, G.W., Echenberg, D.F., Hopewell, P.C. (1987). Tuberculosis in patients with the acquired immunodeficiency syndrome. Am. Rev. Respir. Dis., 23:56-74.

Collins, D.M., and Stephens, D.M. (1991). Identification of an insertion sequence, 1S1081, in Mycobacterium bovis. FEMS Microbiol. Lett. 83:11-16.

Daley, C.L., Small, P.M., Schecter, G.F., Schoolnik, G.K., McAdam, R.A., Jacobs, W.R., and Hopewell, P.C. (1992). An outbreak of tuberculosis with accelerated progression among persons infected with the human immunodeficiency virus. An analysis using restriction-fragment-length-polymorphism. N. Engl. J. Med., 326:231-235.

Devereux, J., Haeberli, P. and Smithies, O. (1984) A comprehensive set of sequence analysis programs for the VAX. Nucl. Acids Res. 12:387-395.

Eiglmeier, K., Honore, N., and Cole, S.T. (1991). Towards the integration of foreign DNA into the chromosome of Mycobacterium leprae. Research in Microbiology, 142:617-622.

Finzel, B.C., Poulos, T.L. and Kraut, J. (1984). Crystal structure of yeast cytochrome C peroxidase at 1.7 A resolution. J. Biol. Chem. 259:13027-13036.

Garnier, T., and Cole, S.T., (1986). Characterization of a bacteriocinogenic plasmid from Clostridium perfringens and molecular genetic analysis of the bacteriocin-encoding gene. J. Bacteriol., 168:1189-1196.

Gayathri Devi, B., Shaila, M.S., Ramakrishnan, T., and Gopinathan, K.P. (1975). The purification and properties of peroxidase in Mycobacterium tuberculosis H37RV and its possible role in the mechanism of action of isonicotinic acid hydrazide. Biochem. J., 149:187-197.

Hermans, P.W.M., van Soolingen, D. and van Embden, J.D.A. (1992). Characterization of a major polymorphic tandem repeat in Mycobacterium tuberculosis and its potential use in the epidemiology of Mycobacterium kansasii and Mycobacterium Ågordonae. J. Bacteriol. 174:4157-4165.

Heym, B. and Cole, S.T. (1992). Isolation and characterization of isoniazid-resistant mutants of Mycobacterium smegmatis and M. aurum. Res. Microbiol., submitted.

Jackett, P.S., Aber, V. and Lowrie, D. (1978). J. Gen Microbiol., 104:37-45.

Kubica, G.P., Jones Jr., W.D., Abbott, V.D., Beam, R.E., Kilburn, J.O., and Cater Jr., J.C. (1966). Differential identification of mycobacteria. I. Tests on catalase activity. Am. Rev. Resp. Dis., 94:400-405.

Kwok et al., S., J. Virol. 61:1690-1694 (1987). Multidrug resistance results from the accumulation of mutations in the genes for distinct drug targets.

Laemmli, U.K., (1970). Cleavage of structural proteins during the assembly of the head of bacteriophage-T4. Nature (London) 227:680-685.

Loewen, P.C., and Stauffer, G.V. (1990). Nucleotide sequence of katG of Salmonella typhimurium LT2 and characterization of its product, hydroperoxidase I. Mol. Gen. Genet. 224:147-151.

Loprasert, S., Negoro, S. and Okada, H. (1988). Thermostable peroxidase from Bacillus stearothermophilus. J. Gen. Microbiol., 134:1971-1976.

Loprasert, S., Negoro, S., and Okada, H. (1989). Cloning, nucleotide sequence, and expression in Escherichia coli of the Bacillus stearotherrmophilus peroxidase gene (perA). J. Bacteriol., 171:4871-4875.

Maniatis, T., Sambrook, J., and Fritsch, E.F. (1989). Molecular cloning. A laboratory manual. Second Edition 1989. Cold Spring Harbor Laboratory Press.

Matsuo, K., Yamaguchi, R., Yamazaki, R.A., Tasaka, H. and Yamada, T. (1988). Cloning and expression of the Mycobacterium bovis BCG gene for extracellular α antigen. J. Bacteriol., 170:3847-3854.

Middlebrook, G. (1954). Isoniazid-resistance and catalase activity of tubercle bacilli. Am. Rev. Tuberc., 69:471-472.

Middlebrook, G., Cohn, M.L., and Schaefer, W.B. (1954). - Studies on isoniazid and tubercle bacilli. III. The isolation, drug-susceptibility, and catalase-testing of tubercle bacilli from isoniazid-treated patients. Am. Rev. Tuberc., 70:852-872.

Mitchison, D.A., Selkon, J.B. and Lloyd, S. (1963). J. Path. Bact. 86:377-386.

Mulvey, M.R., Sorby PA, Triggs-Raine BL and Loewen PC. Gene 73:337-345 (1988).

Orita, M., Iwahana, I., Kanazawa, H., Itayashi, K., and Sekiya, J. (1989). PNAS 86:2766-2770.

Pearson, W., and Lipman, D. (1988). Improved tools for biological sequence comparisons. Proc. Natl. Acad. Sic. USA. 85:2444-2448.

Quemard, A., Lacave, C., and Laneelle, G. (1991). Isoniazid inhibition of mycolic acid synthesis by cell extracts of sensitive and resistant strains of Mycobacterium aurum. Antimicrob. Ag. Chem., 35:1035-1039.

Saiki et al., R. K., Bio/Technology 3:1008-1012 (1985),

Shoeb, H.A., Bowman B.U.J., Ottolenghi, A.C., and Merola, A.J. (1985). Peroxidase-mediated oxidation of isoniazid. Antimicrobial Agents and Chemotherapy, 27:399-403

Shoeb, H.A., Bowman, B.U.J., Ottolenghi, A.C., and Merola, A.S. (1985). Evidence for the generation of active oxygen by isoniazid treatment of extracts of Mycobacterium tuberculosis H37Ra. Antimicrobial Agents and Chemotherapy, 27:404-407.

Sivaraja, M., Goodin, D.B., Smith, M., and Hoffman, B.M., (1989). Identification by ENDOR of Trp191 as the free-radical site in cytochrome c peroxidase Compound Es. Science, 245:738-740.

Snapper, S.B., Lugosi, L., Jekkel, A., Melton, R.E., Kieser, T., Bloom, B.R., and Jacobs, W.R. (1988). Lysogeny and transformation in mycobacteria: stable expression of foreign genes. Proc. Natl. Acad. Sci. USA, 85:6987-6991.

Snapper, S.B., Melton, R.E., Mustafa, S., Kieser, T., and Jacobs, W.R. (1990). Isolation and characterization of efficient plasmid transformation mutants of Mycobacterium smegmatis. Mol. Microbiol., 4:1911-1919.

Snider, D. (1989). Rev. Inf. Dis., S335.

Snider Jr., D.E. and Roper, W.L. (1992). The new tuberculosis. The New Enaland Journal of Medicine, 326:703-705.

Sriprakash, K.S. and Ramakrishnan, T. (1970). Isoniazid-resistant mutants of Mycobacterium tuberculosis H37Rv: Uptake of isoniazid and the properties of NADase inhibitor. J. Gen. Microbiol., 60:125-132.

Staden, R. (1987). Computer handling of sequence projects. In Nucleic acid and protein sequence analysis: A practical approach. Bishop, M.J. and Rawlings, C.J. (eds.) Oxford: IRL Press, pp. 173-217.

Thierry, D., Brisson-Noël, A., Vincént-Levy-Frébault, V., Nguyen, S., Guesdon, J., and Gicquel, B. (1990). Characterization of a Mycobacterium tuberculosis insertion sequence, IS6110, and its application in diagnosis. S. Clin. Microbiol., 28:2668-2673.

Thierry, D., Cave, M.D., Eisenach, K.D., Crawford, S.T., Bates, S.H., Gicquel, B., and Guesdon, J.L. (1990). IS6110, an IS-like element of Mycobacterium tuberculosis complex. Nucleic Acids Res., 18:188.

Triggs-Raine, B.L., Doble, B.W., Mulvey, M.R., Sorby, P.A., and Loewen, P.C. (1988). Nucleotide sequence of kagG, encoding catalase HPI of Escherichia coli. J. Bacteriol., 170:4415-4419.

Wayne, L.G. and Diaz, G.A. (1986). Analyt. Biochem. 157:89-92.

Welinder, K.G. (1991). Bacterial catalase-peroxidases are gene duplicated members of the plant peroxidase superfamily. Biochim. Biophys. Acta 1080:215-220.

Winder, F. and Collins, P. (1968). The effect of isoniazid on nicotinamide nucleotide levels in Mycobacterium bovis, strain BCG. Amer. Rev. Respir. Dis., 97:719-720.

Winder, F. and Collins, P. (1969). The effect of isoniazid on nicotinamide nucleotide concentrations in tubercle bacilli. Amer. Rev. Respir. Dis., 100:101-103.

Winder, F. and Collins, P. (1968). Inhibition by isoniazid of synthesis of mycolic acids in Mycobacterium tuberculosis, J. Gen. Microbiol., 63:41-48.

Youatt, J. (1969). A review of the action of isoniazid. Am. Rev. Respir. Dis., 99:729-749.

Zhang, Y., Garbe, T., and Young, D. (1993). Transformation with katG restores isoniazid-sensitivity in Mycobacterium tuberculosis isolates resistant to a range of drug concentrations. Mol. Microbiol., submitted.

Zhang, Y., and Young, D.B. (1993) Characterization of a variable genetic element from the katG region of Mycobacterium tuberculosis - in preparation.

Zhang, Y., Lathigra, R., Garbe, T., Catty, D., and Young, D. (1991) Genetic analysis of superoxide dismutase, the 23 kilodalton antigen of Mycobacterium tuberculosis. Mol. Microbiol., 5:381-391.

Zhang, Y., Heym, B., Allen, B., Young, D., and Cole, S.T. (1992). The catalase-peroxidase gene and isoniazid resistance of Mycobacterium tuberculosis. Nature. 358:591-593.

Zhang, Y., Garcia, M.J., Lathigra, R., Allen, B., Moreno, C., van Embden, D.A., and Young, D. (1992). Alterations in the superoxide dismutase gene of an isoniazid-resistant strain of Mycobacterium tuberculosis. Infect. Immun., 60:2160-2165.

## Claims

1. A process for the detection of a resistance to an antibiotic in a mycobacterium which comprises detecting a mutation in a gene selected from the group comprising
- the rpoB gene or fragment thereof, which gene or fragment thereof is capable of hybridizing with the sequence shown in Figure 13, and
- the rpSL gene or fragment thereof, which gene or fragment thereof is capable of hybridizing with the Mycobacterium tuberculosis sequence shown in Figure 14.

2. A process for detecting in vitro the presence of nucleic acids of Mycobacterium tuberculosis resistant to isoniazid, wherein the process comprises the steps of:
- contacting said nucleic acids previously made accessible to a probe if required under conditions permitting hybridization;
- detecting any probe that had hybridized to said nucleic acids;
wherein said probe comprises a nucleic acid sequence, which is 2.5 kb EcoRV-KpnI fragment of plasmid pYZ56, and wherein said fragment contains a BamHI cleavage site, wherein isoniazid-resistant Mycobacterium tuberculosis do not contain DNA which hybridises with this fragment.

3. The process as claimed in claim 2, which comprises the steps of:
(A) depositing and fixing nucleic acids of Mycobacterium tuberculosis on a solid support, so as to make the nucleic acids accessible to a probe;
(B) contacting said fixed nucleic acids from step (A) with the probe, as defined in claim 2, under conditions permitting hybridization;
(C) washing said filter resulting from step (B), so as to eliminate any non-hybridized probe; and then
(D) detecting any hybridized probe on said washed filter resulting from step (C).

4. The process of claim 2 or 3 wherein said probe comprises a nucleic acid sequence which encodes a polypeptide of the formula Ala Pro Leu Asn Ser Trp Pro Asp Asn Ala Ser Leu Asp Lys Ala Arg Arg Leu Leu Trp Pro Ser Lys Lys Lys Tyr Gly Lys Lys Leu Ser Trp Ala Asp Leu Ile Val.

5. A process as claimed in any of claims 2 to 4, wherein the probe has a label selected from the group consisting of radioactive, enzymatic, fluorescent, and luminescent labels.

6. The use of the process of any one of claims 2 to 5 for the detection of the presence of Mycobacterium tuberculosis resistant to isoniazid in a bacteria-containing sample suspected of containing Mycobacterium tuberculosis resistant to isoniazid.

7. The use of claim 6, wherein prior to the contacting of said DNA with said probe, said bacteria had been separated from said sample and immobilized on a DNA binding support, which is a nitrocellulose membrane.

8. A nucleic acid probe for detecting Mycobacterium tuberculosis resistant to isoniazid, wherein said probe consists of a 2.5 kb EcoRV-KpnI fragment of plasmid pYZ56, wherein said fragment contains a BamHI cleavage site, or of a part of said fragment which encodes a polypeptide of the formula
APLNSWPDNASLDKARRLLWPSKKKYGKKLSWADLIV.

9. A hybrid duplex molecule consisting essentially of the probe of claim 8 hydrogen bonded to a nucleotide sequence of complementary base sequence.

10. A process for selecting a nucleotide sequence of a Mycobacterium tuberculosis resistant to isoniazid from a group of nucleotide sequences, comprising the step of determining which of said nucleotide sequences hybridizes to a probe as claimed in claim 8 or 9.

11. The process of claim 1 for the detection of resistance to the selected antibiotic which comprises:
- fragmenting the relevant gene or part thereof likely to carry the mutation into a plurality of fragments,
- hybridizing these fragments to a series of labelled oligonucleotide probes recognizing under stringent conditions, all of the parts of the relevant gene of a corresponding control DNA of a strain non-resistant to the corresponding antibiotic,
- and relating the absence of hybridization of at least one of said oligonucleotide probes to any of the DNA fragments of the relevant gene of the mycobacterium under study as evidence of the presence of a mutation and, possibly, of resistance to the corresponding antibiotic, particularly as compared to results obtained upon running the test under the same conditions with the same oligonucleotides on the relevant gene(s) obtained from a strain (strains) not resistant to said antibiotic, wherein said relevant gene is either the rpoB gene or fragment thereof, which gene or fragment thereof is capable of hybridizing with the sequence shown in Figure 13, or the rpsL gene or fragment thereof, which gene or fragment thereof is capable of hybridizing with the Mycobacterium tuberculosis sequence shown in Figure 14.

12. The process of claim 11 which comprises fragmenting by digestion of said relevant gene by selected restriction enzymes.

13. The process of claim 1 which comprises:
- digesting the DNA to be analyzed,
- amplifying the fragments obtained,
- recovering the amplified fragments, and
- separating them from one another according to sizes by causing them to migrate,
- comparing the sizes of the different fragments with those obtained from the DNA(s) of one or several control strains not resistant to the antibiotic, which had been subjected to a similar assay, and
- relating the polymorphism possibly detected to the existence of a mutation in the relevant gene, accordingly to a possible resistance to the corresponding antibiotic of the strain from which the DNA under study had been obtained, wherein said relevant gene is either the rpoB gene or fragment thereof, which gene or fragment thereof is capable of hybridizing with the sequence shown in Figure 13, or the rpsL gene or fragment thereof, which gene or fragment thereof is capable of hybridizing with the Mycobacterium tuberculosis sequence shown in Figure 14.

14. The process of claim 13 which comprises:
- amplifying the fragments by PCR, and
- separating the amplified fragments from one another by causing them to migrate on an electrophoretic gel.

15. A kit for the in vitro diagnostic of the resistance of a bacteria of a mycobacterium genus to isoniazid, **characterized in that** it comprises:
- means for carrying out for a genic amplification of the DNA of the katG gene or of a fragment thereof, which gene or fragment thereof is capable of hybridizing with a 2.5 kb EcoRV-KpnI fragment of plasmid pYZ56,
- means to bring into evidence one or several mutations on the amplification products so obtained, and
- a preparation of control DNA of a katG gene of a strain of said bacteria sensitive to isoniazid or of a fragment thereof.

16. A kit according to claim 15, **characterized in that** further it comprises a control preparation of DNA of the katG gene of an isoniazid-resistant mycobacterium strain.

17. A kit for the in vitro diagnostic of the resistance of a bacteria of a mycobacterium genus to rifampicin or its analogues, **characterized in that** it comprises:
- means for carrying out for a genic amplification of the DNA of the rpoB gene or of the β-sub-unit of the RNA polymerase of said mycobacteria, or of a fragment thereof, which gene or fragment thereof is capable of hybridizing with the sequence shown in Figure 13,
- means to bring into evidence one or several mutations on the amplification products so obtained, and
- a preparation of control DNA of a rpaoB gene coding for the β-sub-unit of the RNA polymerase of a strain of said bacteria sensitive to rifampicin or of a fragment thereof.

18. A kit according to claim 17, **characterized in that** further it comprises a control preparation of a DNA of the rpoB gene of an isoniazid-resistant mycobacterium strain.

19. A kit for the in vitro diagnostics of the resistance of M. tuberculosis to streptomycin, **characterized in that** it includes:
- means for carrying out a genic amplification of the rpsL gene coding for the S12 protein of the small ribosome sub-unit, or fragment thereof, which gene or fragment thereof is capable of hybridizing with the Mycobacterium tuberculosis sequence shown in Figure 14,
- means which enable the bringing of evidence of one or several mutations on the amplification products obtained, and
- a control preparation of a DNA sequence of the rpsL gene coding for the S12 protein of the small sub-unit of the ribosome of a M. tuberculosis strain sensitive to streptomycin.

20. A kit according to claim 19, **characterized in that** it includes a control preparation of a DNA sequence of a rpsL gene coding for the S12 protein of the small sub-unit of the ribosome of a strain of M. tuberculosis resistant to streptomycin.

21. A nucleotide sequence which is the 263 base sequence as described in Figure 15.

22. A nucleotide sequence according to claim 21, comprising a mutation of codon 43, AAG being mutated into AGG.

23. A nucleotide sequence which is the 3447 base sequence as described in Figure 12, or a portion thereof, said portion being :
- the sequence illustrated in Figure 11A ;
- the 710 base pair fragment obtainable by amplification of the sequence illustrated in Figure 12 with the primers CAGGACGTCGAGGCGATCAC and AACGACGACGTGGGCAGCGT;
- the nucleotide sequence extending from nucleotides 1195 to 1293 in Figure 12, encoding the amino acid sequence illustrated in Figure 11B.

24. A nucleotide sequence which is the 432 base sequence described in Figure 13, or a portion thereof, said portion being:
- a sequence including the codons 400 - 450 of the rpoB gene ;
- the nucleotide sequence extending from nucleotides 169 to 267 in Figure 13, encoding the amino acid sequence illustrated in Figure 11B.

25. A nucleotide sequence according to claims 23 or 24, comprising a mutation localized in the region 400-450.

26. A nucleotide sequence according to claim 25, comprising a mutation of codon 425.

27. Nucleic acid sequence comprising a 2.5 kb EcoRV-KpnI fragment of plasmid pYZ56.

28. Nucleic acid sequence according to claim 27 comprising a 4.5 kb KpnI fragment of plasmid pYZ55, wherein said fragment contains a BamHI cleavage site.

29. Use of a nucleic acid sequence comprising the sequence of any of claims 21 to 28 for the detection of antibiotic resistance in Mycobacteria.

## Patentansprüche

1. Verfahren zum Nachweis einer Resistenz gegen ein Antibiotikum in einem Mycobacterium, welches umfasst, eine Mutation in einem Gen nachzuweisen, das ausgewählt ist aus der Gruppe, umfassend
das rpoB-Gen oder ein Fragment davon, wobei das Gen oder das Fragment davon in der Lage ist, mit der in Figur 13 gezeigten Sequenz zu hybridisieren, und
das rpSL-Gen oder ein Fragment davon, wobei das Gen oder das Fragment davon in der Lage ist, mit der in Figur 14 gezeigten Mycobacterium tuberculosis-Sequenz zu hybridisieren.

2. Verfahren zum in vitro-Nachweis der Anwesenheit von Nukleinsäuren eines Mycobacterium tuberculosis, das gegenüber Isoniazid resistent ist, wobei das Verfahren die folgenden Schritten umfasst:
- Inkontaktbringen der Nukleinsäuren, die vorab, sofern erforderlich, für eine Sonde zugänglich gemacht wurden, unter Bedingungen, die eine Hybridisierung ermöglichen,
- Nachweisen von jeglicher Sonde, die mit den Nukleinsäuren hybridisiert hat;
wobei die Sonde eine Nukleinsäuresequenz umfasst, die ein 2,5 kb-EcoRV-KpnI-Fragment von Plasmid pYZ56 ist, und wobei das Fragment BamHI-Spaltstelle enthält, wobei ein Isoniazid-resistentes Mycobacterium tuberculosis keine DNA enthält, die mit diesem Fragment hybridisiert ist.

3. Verfahren nach Anspruch 2, das die folgenden Schritte umfasst:
(A) Abscheiden und Fixieren von Nukleinsäuren von Mycobacterium tuberculosis auf einem festen Träge, um die Nukleinsäuren für eine Sonde zugänglich zu machen,
(B) Inkontaktbringen der fixierten Nukleinsäuren aus Schritt (A) mit der Sonde, wie in Anspruch 2 definiert, unter Bedingungen, die eine Hybridisierung erlauben,
(C) Waschen des aus Schritt (B) resultierenden Filters, um jegliche nicht hybridisierte Sonde zu entfernen, und dann
(D) Nachwesen jeglicher hybridisierter Sonde auf dem aus Schritt (C) resultierenden gewaschenen Filter.

4. Verfahren nach Ansrpuch 2 oder 3, wobei die Sonde eine Nukleinsäuresequenz umfasst, die ein Polypeptid der Formel Ala Pro Leu Asn Ser Trp Pro Asp Asn Ala Ser Leu Asp Lys Ala Arg Arg Leu Leu Trp Pro Ser Lys Lys Lys Tyr Gly Lys Lys Leu Ser Tro Ala Asp Leu Ile Val kodiert.

5. Verfahren nach einem der Ansprüche 2 bis 4, wobei die Sonde eine Markierung, ausgewählt aus der Gruppe, bestehend aus radioaktiven, enzymatischen, fluoreszierenden und luminiszierenden Markierungen, aufweist.

6. Verwendung des Verfahrens nach einem der Ansprüche 2 bis 5 für den Nachweis der Anwesenheit von gegenüber Isoniazid resistentem Mycobacterium tuberculosis in einer Bakterien enthaltenden Probe, von der vermutet wird, dass sie gegenüber Isoniazid resistentes Mycobacterium tuberculosis enthält.

7. Verwendung nach Anspruch 6, wobei vor dem Inkontaktbringen der DNA mit der Sonde die Bakterien von der Probe abgetrennt und auf einem DNA-bindenden Träger, der eine Nitrocellulosemembran ist, immobilisiert worden sind.

8. Nukleinsäuresonde zum Nachweisen von gegenüber Isoniazid resistentem Mycobacterium tuberculosis, wobei die Sonde aus einem 2,5 kb-EcoRV-KpnI-Fragment von Plasmid pYZ56, wobei das Fragment eine BamHI-Spaltstelle enthält, oder aus einem Teil des genannten Fragments, der ein Polypeptid der Formel APLNSWPDNASLDKARRLLWPSKKKYGKKLSWADLIV, besteht.

9. Hybrides Doppelstrangmolekül, bestehend im Wesentlichen aus der Sonde von Anspruch 8, die über Wasserstoffbrücken an eine Nukleotidsequenz von komplementärer Basensequenz gebunden ist.

10. Verfahren zum Selektieren einer Nukleotidsequenz eines gegenüber Isoniazid resistenten Mycobacterium tuberkulosis aus einer Gruppe von Nukleotidsequenzen, umfassend den Schritt, zu Bestimmen, welche der Nukleotidsequenzen mit einer Sonde, wie in Anspruch 8 oder 9 beansprucht, hybridisiert.

11. Verfahren nach Anspruch 1 für den Nachweis einer Resistenz gegenüber dem ausgewählten Antibiotikum, welches umfasst:
- Fragment des relevanten Gens oder eines Teils davon, der wahrscheinlich die Mutation trägt, zu einer Mehrzahl von Fragmenten,
- Hybridisieren dieser Fragmente mit einer Reihe von markierten Oligonukleotidsonden die unter stringenten Bedingungen sämtliche der Teile des relevanten Gens eines entsprechenden Kontroll-DNA von einem gegenüber dem entsprechenden Antibiotikum nicht resistenten Stamm erkennen;
- und In-Beziehung-Bringen des Fehlens von Hybridisierung von mindestens einer der Oligonukleotidsäuren mit einerm der DNA-Fragmente des relevanten Gens des untersuchten Mycobacteriums als Nachweis für die Anwesenheit einer Mutation und möglicherweise für eine Resistenz gegenüber dem entsprechenden Antibiotikum insbesondere im Vergleich mit Ergebnissen, die erhalten werden, wenn man den Test unter den gleichen Bedingungen mit den gleichen Oligonukleotiden an dem oder den relevanten Gen(en), erhalten von einem Stamm (Stämme), der gegenüber dem Antibiotikum nicht resistent ist, durchgeführt hat, wobei das relevante Gen entweder das rpoB-Gen oder ein Fragment davon ist, wobei das Gen oder das Fragment davon in der Lage ist, mit der in Figur 13 gezeigten Frequenz zu hybridisieren, oder das rpsL-Gen oder ein Fragment davon ist, wobei das Gen oder das Fragment davon in der Lage ist, mit der in Figur 14 gezeigten Mycobacterium tuberculosis-Sequenz zu hybridisieren.

12. Verfahren nach Anspruch 11, welches ein Fragmentieren durch einen Verdau des relevanten Gens durch ausgewählte Restriktionsenzyme umfasst.

13. Verfahren nach Anspruch 1, welches umfasst:
- Verdauen der zu analysierenden DNA,
- Amplifizieren der erhaltenen Fragmente,
- Gewinnen der amplifizierten Fragmente und
- Auftrennen derselben gemäß den Größen, indem man sie wandern lässt,
- Vergleichen der Größen der verschiedenen Fragmente mit jenen, die von der oder den DNA(s) von einem oder mehreren Kontrollstämmen, die gegenüber dem Antibiotikum nicht resistent sind, erhalten wurden und die einem ähnlichen Assay unterworfen worden waren, und
- In-Beziehung-Bringen des möglicherweise nachgewiesenen Polymorphismus mit der Existenz einer Mutation in dem relevanten Gen, dementsprechend mit einer möglichen Resistenz des Stamms, von dem die untersuchte DNA erhalten worden ist, gegenüber dem entsprechenden Antibiotikum, wobei das relevante Gen entweder das rpoB-Gen oder ein Fragment davon ist, wobei das Gen oder das Fragment davon in der Lage ist, mit der in Figur 13 gezeigten Frequenz zu hybridisieren, oder das rpsL-Gen oder ein Fragment davon ist, wobei das Gen oder das Fragment davon in der Lage ist, mit der in Figur 14 gezeigten Mycobacterium tuberculosis-Sequenz zu hybridisieren.

14. Verfahren nach Anspruch 13, welches umfasst:
- Amplifizieren der Fragmente durch PCR und
- Auftrennen der amplifizierten Fragmente, indem man sie auf einen E-lektrophoresesegel wandern lässt.

15. Kit für die in vitro-Diagnose der Resistenz eines Bakteriums einer Mycobacterium-Gattung gegenüber Isoniazid, **dadurch gekennzeichnet, dass** er umfasst:
- Mittel zum Ausführen einer Genamplifikation der DNA des katG-Gens oder eines Fragments davon, wobei das Gen oder Fragment davon in der Lage ist, mit einem 2,5 kb-EcoRV-KpnI-Fragment von Plasmid pYZ56 zu hybridisieren,
- Mittel zum Nachweisen einer oder mehrerer Mutationen bei den so erhaltenen Amplifikationsprodukten und
- eine Präparation von Kontroll-DNA eine katG-Gens eines Stammes des Bakteriums, der gegenüber Isoniazid empfindlich ist, oder eines Fragments davon.

16. Kit nach Anspruch 15, **dadurch gekennzeichnet, dass** er ferner eine Kontroll-präparation einer DNA des katG-Gens eines Isoniazid-resistenten Mycobacterium-Stammes enthält.

17. Kit für die in vitro-Diagnose der Resistenz eines Bakteriums einer Mycobacterium-Gattung gegenüber Rifampicin oder dessen Analoga, **dadurch gekennzeichnet, dass** er umfasst:
- Mittel zum Ausführen einer Genamplifikation der DNA des rpoB-Gens oder der β-Untereinheit der RNA-Polymerase des Mycobacteriums oder eines Fragments davon, wobei das Gen oder das Fragment davon in der Lage ist, mit der in Figur 13 gezeigten Sequenz zu hybridisieren,
- Mittel zum Nachweisen einer oder mehrerer Mutationen bei den so erhaltenen Amplifikationsprodukten und
- Eine Präparation von Kontroll-DNA eines rpoB-Gens, das die β-Untereinheit der RNA-Polymerase von einem Stamm des Bakteriums, der gegenüber Rifampicin empfindlich ist, kodiert, oder von einem Fragment davon.

18. Kit nach Anspruch 17, **dadurch gekennzeichnet, dass** er ferner eine Kontroll-präparation von einer DNA des rpoB-Gens eines Isoniazid-resistenten Mycobacterium-Stammes umfasst.

19. Kit für die in vitro-Diagnose der Resistenz von M. tuberculosis gegenüber Streptomycin, **dadurch gekennzeichnet, dass** er umfasst:
- Mittel zum Ausführen einer Genamplifikation des rpsL-Gens, das das S12-Protein der kleinen Ribosomenuntereinheit kodiert, oder eines Fragments davon, wobei das Gen oder das Fragment davon in der Lage ist, mit der in Figur 14 gezeigten Mycobacterium tuberculosis-Sequenz zu hybridisieren,
- Mittel, die den Nachweis einer oder mehrerer Mutationen bei den erhaltenen Amplifikationsprodukten ermöglichen, und
- Eine Kontroll-Präparation einer DNA-Sequenz des rpsL-Gens, das das S12-Protein der kleinen Untereinheit des Ribosoms eines gegenüber Streptomycin empfindlichen M. tuberculosis-Stamms kodiert.

20. Kit nach Anspruch 19, **dadurch gekennzeichnet, dass** er eine Kontrollpräparation einer DNA-Sequenz eines rpsL-Gens, das das S12-Protein der kleinen Untereinheit des Ribosoms eines gegenüber Streptomycin resistenten M. tuberculosis-Stamms kodiert, umfasst.

21. Nukleotidsequenz, die die 263 Basen-Sequenz, wie in Figur 15 beschrieben, ist.

22. Nukleotidsequenz nach Anspruch 21, umfassend eine Mutation des Kodons 43, wobei AAG zu AGG mutiert ist.

23. Nukleotidsequenz, die die 3447 Basen-Sequenz, wie in Figur 12 beschrieben, ist, oder ein Abschnitt davon, der genannte Abschnitt ist:
- die in Figur 11A abgebildete Sequenz;
- das 710 Basenpaar-Fragment, erhältlich durch Amplifizierung der in Figur 12 abgebildeten Sequenz, mit den Primern CAGGACGTCGAGGCGATCAC und AACGACGACGTGGCCAGCGT;
- die Nukleotidsequenz, die sich von den Nukleotiden 1195 bis 1293 in Figur 12 erstreckt, die die in Figur 11B abgebildete Aminosäuresequenz kodiert.

24. Nukleotidsequenz, die die 432 Basen-Sequenz, wie in Figur 13 beschrieben, ist, oder ein Abschnitt davon, der genannte Abschnitt ist:
- eine Sequenz, die die Kodons 400-450 des rpoB-Gens einschließt;
- die Nukleotidsequenz, die sich von den Nukleotiden 169 bis 267 in Figur 13 erstreckt, die die in Figur 11B abgebildete Aminosäuresequenz kodiert.

25. Nukleotidsequenz nach Anspruch 23 oder 24, umfassend eine in dem Bereich 400-450 lokalisierte Mutation.

26. Nukleotidsequenz nach Anspruch 25, umfassend eine Mutation des Kodons 425.

27. Nukleinsäuresequenz, umfassend ein 2,5 kb EcoRV-KpnI-Fragment von Plasmid pYZ56.

28. Nukleinsäuresequenz nach Anspruch 27, umfassend ein 4,5 kb KpnI-Fragment von Plasmid pYZ55, wobei das Fragment eine BamHI-Schnittstelle enthält.

29. Verwendung einer Nukleinsäuresequenz, umfassend die Sequenz nach einem der Ansprüche 21 bis 28, für den Nachweis einer Antibiotikaresistenz in Mycobakterien.

## Revendications

1. Procédé de détection de résistance à un antibiotique dans une mycobactérie qui comprend la détection d'une mutation dans un gène choisi dans le groupe comprenant
le gène rpoB ou un fragment de celui-ci, lequel gène ou fragment est capable de s'hybrider à la séquence présentée à la Figure 13,
et le gène rpsL ou un fragment de celui-ci, lequel gène ou fragment est capable de s'hybrider à la séquence de Mycobacterium tuberculosis présentée à la Figure 14.

2. Procédé pour la détection *in vitro* de la présence d'acides nucléiques de Mycobacterium tuberculosis résistant à l'isoniazide, où le procédé comprend les étapes
- de mise en contact desdits acides nucléiques, prétraités si besoin est pour les rendre accessible à une sonde dans un milieu permettant l'hybridation;
- la détection de toute sonde qui s'est hybridée auxdits acides nucléiques;
où ladite sonde comprend une séquence d'acide nucléique, qui correspond à un fragment EcoRV-KpnI de 2,5 kb du plasmide pYZ56, et où ledit fragment contient un site de clivage BamHI, où Mycobacterium tuberculosis résistant à l'isoniazide ne contient pas d'ADN qui s'hydride avec ce fragment.

3. Procédé selon la revendication 2, qui comprend les étapes
(A) de dépôt et de fixation d'acides nucléiques de Mycobacterium tuberculosis à un support solide, de manière à rendre les acides nucléiques accessibles à une sonde;
(B) de mise en contact desdits acides nucléiques issus de l'étape (A) avec la sonde, telle que définie dans la revendication 2, dans un milieu permettant l'hybridation;
(C) de lavage dudit filtre résultant de l'étape (B), de manière à éliminer toute sonde sous forme non hybridée; et ensuite
(D) de détection de toute sonde sous forme hybridée sur ledit filtre lavé résultant de l'étape (C).

4. Procédé selon la revendication 2 ou 3 dans lequel ladite sonde comprend une séquence d'acide nucléique qui code pour un polypeptide de séquence Ala Pro Leu Asn Ser Trp Pro Asp Asn Ala Ser Leu Asp Lys Ala Arg Arg Leu Leu Trp Pro Ser Lys Lys Lys Tyr Gly Lys Lys Leu Ser Trp Ala Asp Leu Ile Val.

5. Procédé selon l'une quelconque des revendications 2 à 4, dans lequel la sonde comporte un marqueur choisi dans le groupe constitué de marqueurs luminescents, fluorescents, enzymatiques et radioactifs.

6. Utilisation du procédé selon l'une quelconque des revendications 2 à 5 pour détecter la présence de Mycobacterium tuberculosis résistant à l'isoniazide dans un échantillon contenant des bactéries présumé contenir une souche de Mycobacterium tuberculosis résistante à l'isoniazide.

7. Utilisation selon la revendication 6, dans laquelle préalablement à la mise en contact dudit ADN avec ladite sonde, lesdites bactéries se trouvent déjà séparées dudit échantillon et immobilisées sur un support de fixation d'ADN, qui est une membrane en nitrocellulose.

8. Sonde d'acide nucléique pour détecter une souche de Mycobacterium tuberculosis résistante à l'isoniazide, dans laquelle ladite sonde consiste en un fragment EcoRV-KpnI de 2,5 kb du plasmide pYZ56, dans lequel ledit fragment contient un site de clivage BamHI, ou en une partie dudit fragment qui code pour un polypeptide de formule APLNSWPDNASLDKARRLLWPSKKKYGKKLSWADLIV.

9. Molécule duplex hybride consistant essentiellement en la sonde selon la revendication 8, liée par des liaisons hydrogène à une séquence nucléotidique à bases complémentaires.

10. Procédé de sélection d'une séquence nucléotidique de Mycobacterium tuberculosis résistant à l'isoniazide dans un groupe de séquence nucléotidiques, comprenant l'étape consistant à déterminer laquelle desdites séquences nucléotidiques s'hybride à une sonde selon la revendication 8 ou 9.

11. Procédé selon la revendication 1 pour la détection de résistance à l'antibiotique sélectionné qui comprend :
- la fragmentation du gène d'intérêt ou d'une partie de celui-ci, susceptible de porter la mutation, en une pluralité de fragments,
- l'hybridation de ces fragments à une série de sondes oligonucléotidiques marquées, reconnaissant, en milieu stringent, l'ensemble des parties du gène d'intérêt d'un ADN témoin correspondant, dérivé d'une souche non résistante à l'antibiotique correspondant,
- et l'interprétation de l'absence d'hybridation d'au moins une desdites sondes oligonucléotidiques à l'un quelconque des fragments d'ADN du gène d'intérêt de la mycobactérie à l'étude comme étant une indication de la présence d'une mutation et, éventuellement, d'une résistance à l'antibiotique correspondant, en particulier en se référant aux résultats obtenus en effectuant l'essai dans les mêmes conditions avec les mêmes oligonucléotides sur le ou les gènes d'intérêt obtenus à partir d'une ou plusieurs souches non résistantes audit antibiotique, dans lequel ledit gène d'intérêt est soit le gène rpoB ou un fragment de celui-ci, lequel gène ou fragment est capable de s'hybrider à la séquence présentée à la Figure 13, soit le gène rpsL ou fragment de celui-ci, lequel gène ou fragment est capable de s'hybrider à la séquence de Mycobacterium tuberculosis présentée à la Figure 14.

12. Procédé selon la revendication 11 qui comprend la fragmentation par digestion dudit gène d'intérêt par des enzymes de restriction sélectionnées.

13. Procédé selon la revendication 1 qui comprend
- la digestion de l'ADN devant être analysé,
- l'amplification des fragments résultants,
- la récupération des fragments amplifiés, et
- la séparation des fragments entre eux d'après la taille en les soumettant à migration,
- la comparaison des tailles des différents fragments à celles observées avec un ou plusieurs ADN d'une ou de plusieurs souches témoins non résistantes à l'antibiotique, soumises au préalable à un essai semblable, et
- l'assimilation de tout polymorphisme éventuellement détecté à l'existence d'une mutation au sein du gène d'intérêt, et par conséquent à l'existence d'une éventuelle résistance à l'antibiotique correspondant chez la souche dont provient l'ADN à l'étude, dans lequel ledit gène d'intérêt est soit le gène rpoB ou un fragment de celui-ci, lequel gène ou fragment est capable de s'hybrider à la séquence présentée à la Figure 13, soit le gène rpsL ou un fragment de celui-ci, lequel gène ou fragment est capable de s'hybrider à la séquence de Mycobacterium tuberculosis présentée à la Figure 14.

14. Procédé selon la revendication 13 qui comprend :
- l'amplification des fragments par PCR, et
- la séparation des fragments amplifiés entre eux en les soumettant à migration sur un gel d'électrophorèse.

15. Kit diagnostique pour déceler *in vitro* la résistance d'une bactérie du genre Mycobacterium à l'isoniazide, **caractérisée en ce qu'**il comprend :
- des moyens pour réaliser une amplification génique de l'ADN du gène katG ou d'un fragment de celui-ci, lequel gène ou fragment est capable de s'hybrider à un fragment EcoRV-KpnI de 2,5 kb du plasmide pYZ56,
- des moyens pour mettre en évidence une ou plusieurs mutations au niveau des produits d'amplifications ainsi obtenus, et
- une préparation d'ADN témoin d'un gène katG venant d'une souche de ladite bactérie sensible à l'isoniazide ou d'un fragment de celui-ci.

16. Kit selon la revendication 15, **caractérisé en ce qu'**il comprend en outre une préparation témoin d'ADN du gène katG venant d'une souche de Mycobacterium résistante à l'isoniazide.

17. Kit diagnostique pour déceler *in vitro* la résistance d'une bactérie du genre Mycobacterium à la rifampicine ou à ses analogues, **caractérisé en ce qu'**il comprend :
- des moyens pour réaliser une amplification génique de l'ADN du gène rpoB ou de la sous-unité β de l'ARN- polymérase de ladite mycobactérie, ou d'un fragment de celui-ci, lequel gène ou fragment est capable de s'hybrider à la séquence présentée à la Figure 13,
- des moyens pour mettre en évidence une ou plusieurs mutations au niveau des produits d'amplification ainsi obtenus, et
- une préparation d'ADN témoin d'un gène rpoB codant pour la sous-unité β de l'ARN-polymérase venant d'une souche de ladite bactérie sensible à la rifampicine ou d'un fragment de celui-ci.

18. Kit selon la revendication 17, **caractérisé en ce qu'**il comprend en outre une préparation témoin d'un ADN du gène rpoB dérivé d'une souche de mycobactérie résistante à l'isoniazide.

19. Kit diagnostique in vitro pour déceler la résistance de M. tuberculosis à la streptomycine, **caractérisé en ce qu'**il comprend :
- des moyens pour réaliser une amplification génique du gène rpsL codant pour la protéine S12 de la petite sous-unité ribosomique, ou d'un fragment de celui-ci, lequel gène ou fragment est capable de s'hybrider à la séquence de Mycobacterium tuberculosis présentée à la Figure 14,
- des moyens permettant la mise en évidence d'une ou de plusieurs mutations au niveau des produits d'amplification obtenus. et
- une préparation témoin d'une séquence d'ADN du gène rpsL codant pour la protéine S12 de la petite sous- unité ribosomique d'une souche de M. tuberculosis sensible à la streptomycine.

20. Kit selon la revendication 19, **caractérisé en ce qu'**il comprend une préparation témoin d'une séquence d'ADN d'un gène rpsL codant pour la protéine S12 de la petite sous-unité ribosomique d'une souche de M. tuberculosis résistante à la streptomycine.

21. Séquence nucléotidique qui est la séquence de 263 bases décrite à la Figure 15.

22. Séquence nucléotidique selon la revendication 21, comprenant une mutation sur le codon 43, AAG étant muté en AGG.

23. Séquence nucléotidique qui est la séquence de 3447 bases décrite à la Figure 12, ou une portion de celle-ci, ladite portion étant
- la séquence illustrée à la figure 11A ;
- Le fragment de 710 paires de base qui peut être obtenu par amplification de la séquence illustrée à la figure 12 à l'aide des amorces CAGGACGTCGAGGCGATCAC et AACGACGACGTGGCCAGCGT;
- La séquence nucléotidique s'étendant des nucléotides 1195 à 1293 à la figure 12, codant pour la séquence en acides aminés illustrée à la figure 11B.

24. Séquence nucléotidique qui est la séquence de 432 bases décrite à la Figure 13, ou une portion de celle-ci, ladite portion étant :
- Une séquence incluant les codons 400-450 du gène rpoB ;
- La séquence nucléotidique s'étendant des nucléotides 169 à 267 à la figure 13, codent pour la séquence en acides aminés illustrée à la figure 11B.

25. Séquence nucléotidique selon la revendication 23 ou 24, comprenant une mutation située dans la région 400 à 450.

26. Séquence nucléotidique selon la revendication 25, comprenant une mutation sur le codon 425.

27. Séquence d'acide nucléotidique comprenant un fragment EcoR-KpnI de 2,5 kb du plasmide pYZ56.

28. Séquence d'acide nucléique selon la revendication 27 comprenant un fragment KpnI de 4,5 kb du plasmide pYZ55, dans laquelle ledit fragment contient un site de clivage BamHI.

29. Utilisation d'une séquence d'acide nucléique comprenant la séquence de l'une quelconque des revendications 21 à 28 pour la détection de résistance aux antibiotiques chez les mycobactéries.
